# EUROPEAN PATENT SPECIFICATION

(11) **EP 2 664 340 B1**
(45) Date of publication and mention of the grant of the patent: **12.02.2020**
(21) Application number: 13176325.2
(22) Date of filing: 21.06.2006
(51) Int. Cl.: A61K 9/00, A61K 47/42, A61P 19/02, A61P 29/00, A61K 38/20, A61K 39/44

(54) **A direct drug delivery system based on thermally responsive biopolymers**
System zur direkten Heilmittelverabreichung auf der basis wärmegesteuerter Biopolymere
Système d'administration directe de médicaments à base de biopolymères thermiquement sensibles

(30) Priority: 24.06.2005 US 693966 P
(43) Date of publication of application: 20.11.2013
(62) Divisional of application: 06785402.6
(73) Proprietor: DUKE UNIVERSITY, Durham NC 27707 (US)
(72) Inventor: Setton, Lori A., Malvern, PA 19355 (US); Chilkoti, Ashutosh, Durham, NC 27701 (US); Kraus, Virginia B., Hillsborough, NC 27278 (US); Betre, Helawe, Malvern, PA 19355 (US); Dreher, Matthew R., Malvern, PA 19355 (US)
(74) Representative: Cooley (UK) LLP

(56) References cited:
- WO-A1-00/56774
- US-A- 5 972 880
- US-B2- 6 852 834
- MEYER D E ET AL: "Drug targeting using thermally responsive polymers and local hyperthermia", JOURNAL OF CONTROLLED RELEASE, ELSEVIER, AMSTERDAM, NL, vol. 74, no. 1-3, 6 July 2001 (2001-07-06) , pages 213-224, XP027296085, ISSN: 0168-3659 [retrieved on 2001-07-06]
- DAN E. MEYER ET AL: "Genetically Encoded Synthesis of Protein-Based Polymers with Precisely Specified Molecular Weight and Sequence by Recursive Directional Ligation: Examples from the Elastin-like Polypeptide System", BIOMACROMOLECULES, vol. 3, no. 2, 1 March 2002 (2002-03-01), pages 357-367, XP055067102, ISSN: 1525-7797, DOI: 10.1021/bm015630n
- CARON J P ET AL: "CHONDROPROTECTIVE EFFECT OF INTRAARTICULAR INJECTIONS OF INTERLEUKIN-1 RECEPTOR ANTAGONIST IN EXPERIMENTAL OSTEOARTHRITIS", ARTHRITIS & RHEUMATISM, JOHN WILEY & SONS, INC, US, vol. 39, no. 9, 1 September 1996 (1996-09-01), pages 1535-1544, XP000674703, ISSN: 0004-3591
- Betre, Helawe: "Controlled intra-articular drug delivery system based on thermally responsive biopolymers", ProQuest® Dissertations & Theses: The Sciences and Engineering Collection , 10 July 2005 (2005-07-10), XP008157979, Retrieved from the Internet: URL:http://gradworks.umi.com/31/90/3190635 .html [retrieved on 2012-11-15]

### SEQUENCE LISTING

<110> Duke University
<120> A DIRECT DRUG DELIVERY SYSTEM BASED ON THERMALLY RESPONSIVE BIOPOLYMERS
<130> PHAS-004/00WO
<140> EP 06785402.6
   <141> 2006-06-21
<140> PCT/US2006/024427
   <151> 2006-06-21
<150> US 60/693,966
   <151> 2005-06-24
<160> 25
<170> PatentIn version 3.3
<210> 1
   <211> 450
   <212> PRT
   <213> Artificial
<220>
   <223> Thermosensitive bioelastic polymer sequence
<400> 1
   Val Pro Gly Val Gly Val Pro Gly Val Gly Val Pro Gly Val Gly Val
   1               5                   10                  15
   Pro Gly Val Gly Val Pro Gly Val Gly Val Pro Gly Val Gly Val Pro
               20                  25                  30
   Gly Val Gly Val Pro Gly Val Gly Val Pro Gly Val Gly Val Pro Gly
           35                  40                  45
   Val Gly Val Pro Gly Val Gly Val Pro Gly Val Gly Val Pro Gly Val
       50                  55                  60
   Gly Val Pro Gly Val Gly Val Pro Gly Val Gly Val Pro Gly Val Gly
   65                  70                  75                  80
   Val Pro Gly Val Gly Val Pro Gly Val Gly Val Pro Gly Val Gly Val
                   85                  90                  95
   Pro Gly Val Gly Val Pro Gly Val Gly Val Pro Gly Val Gly Val Pro
               100                 105                 110
   Gly Val Gly Val Pro Gly Val Gly Val Pro Gly Val Gly Val Pro Gly
           115                 120                 125
   Val Gly Val Pro Gly Val Gly Val Pro Gly Val Gly Val Pro Gly Val
       130                 135                 140
   Gly Val Pro Gly Val Gly Val Pro Gly Val Gly Val Pro Gly Val Gly
   145                 150                 155                 160
   Val Pro Gly Val Gly Val Pro Gly Val Gly Val Pro Gly Val Gly Val
                   165                 170                 175
   Pro Gly Val Gly Val Pro Gly Val Gly Val Pro Gly Val Gly Val Pro
               180                 185                 190
   Gly Val Gly Val Pro Gly Val Gly Val Pro Gly Val Gly Val Pro Gly
           195                 200                 205
   Val Gly Val Pro Gly Val Gly Val Pro Gly Val Gly Val Pro Gly Val
       210                 215                 220
   Gly Val Pro Gly Val Gly Val Pro Gly Val Gly Val Pro Gly Val Gly
   225                 230                 235                 240
   Val Pro Gly Val Gly Val Pro Gly Val Gly Val Pro Gly Val Gly Val
                   245                 250                 255
   Pro Gly Val Gly Val Pro Gly Val Gly Val Pro Gly Val Gly Val Pro
               260                 265                 270
   Gly Val Gly Val Pro Gly Val Gly Val Pro Gly Val Gly Val Pro Gly
           275                 280                 285
   Val Gly Val Pro Gly Val Gly Val Pro Gly Val Gly Val Pro Gly Val
       290                 295                 300
   Gly Val Pro Gly Val Gly Val Pro Gly Val Gly Val Pro Gly Val Gly
   305                 310                 315                 320
   Val Pro Gly Val Gly Val Pro Gly Val Gly Val Pro Gly Val Gly Val
                   325                 330                 335
   Pro Gly Val Gly Val Pro Gly Val Gly Val Pro Gly Val Gly Val Pro
               340                 345                 350
   Gly Val Gly Val Pro Gly Val Gly Val Pro Gly Val Gly Val Pro Gly
           355                 360                 365
   Val Gly Val Pro Gly Val Gly Val Pro Gly Val Gly Val Pro Gly Val
       370                 375                 380
   Gly Val Pro Gly Val Gly Val Pro Gly Val Gly Val Pro Gly Val Gly
   385                 390                 395                 400
   Val Pro Gly Val Gly Val Pro Gly Val Gly Val Pro Gly Val Gly Val
                   405                 410                 415
   Pro Gly Val Gly Val Pro Gly Val Gly Val Pro Gly Val Gly Val Pro
               420                 425                 430
   Gly Val Gly Val Pro Gly Val Gly Val Pro Gly Val Gly Val Pro Gly
           435                 440                 445
   Val Gly
       450
<210> 2
   <211> 600
   <212> PRT
   <213> Artificial
<220>
   <223> Thermosensitive bioelastic polymer sequence
<210> 3
   <211> 110
   <212> PRT
   <213> Artificial
<220>
   <223> Thermosensitive bioelastic polymer sequence
<400> 3
   Val Pro Gly Val Gly Val Pro Gly Val Gly Val Pro Gly Val Gly Val
    1               5                  10                  15
   Pro Gly Val Gly Val Pro Gly Val Gly Val Pro Gly Val Gly Val Pro
               20                  25                  30
   Gly Lys Gly Val Pro Gly Lys Gly Val Pro Gly Lys Gly Val Pro Gly
           35                  40                  45
   Lys Gly Val Pro Gly Lys Gly Val Pro Gly Lys Gly Val Pro Gly Lys
       50                  55                  60
   Gly Val Pro Gly Lys Gly Val Pro Gly Lys Gly Val Pro Gly Lys Gly
    65                  70                  75                  80
   Val Pro Gly Lys Gly Val Pro Gly Lys Gly Val Pro Gly Lys Gly Val
                   85                  90                  95
   Pro Gly Lys Gly Val Pro Gly Lys Gly Val Pro Gly Lys Gly
               100                 105                 110
<210> 4
   <211> 5
   <212> PRT
   <213> Artificial
<220>
   <223> Bioelastic polymer unit sequence
<220>
   <221> MISC_FEATURE
   <222> (4)..(4)
   <223> Xaa can be any naturally occurring amino acid other than proline
<400> 4
   Val Pro Gly Xaa Gly
    1               5
<210> 5
   <211> 40
   <212> PRT
   <213> Artificial
<220>
   <223> Bioelastic polymer sequence
<400> S
<210> 6
   <211> 4
   <212> PRT
   <213> Artificial
<220>
   <223> Bioelastic polymer unit sequence
<400> 6
   Val Pro Gly Gly
   1
<210> 7
   <211> 4
   <212> PRT
   <213> Artificial
<220>
   <223> Bioelastic polymer unit sequence
<400> 7
   Gly Gly Val Pro
   1
<210> 8
   <211> 4
   <212> PRT
   <213> Artificial
<220>
   <223> Bioelastic polymer unit sequence
<400> 8
   Gly Gly Phe Pro
   1
<210> 9
   <211> 4
   <212> PRT
   <213> Artificial
<220>
   <223> Bioelastic polymer unit sequence
<400> 9
   Gly Gly Ala Pro
   1
<210> 10
   <211> 5
   <212> PRT
   <213> Artificial
<220>
   <223> Bioelastic polymer unit sequence
<400> 10
   Val Pro Gly Val Gly
   1 5
<210> 11
   <211> 5
   <212> PRT
   <213> Artificial
<220>
   <223> Bioelastic polymer unit sequence
<400> 11
   Gly Val Gly Val Pro
   1 5
<210> 12
   <211> 5
   <212> PRT
   <213> Artificial
<220>
   <223> Bioelastic polymer unit sequence
<400> 12
   Gly Lys Gly Val Pro
   1 5
<210> 13
   <211> 5
   <212> PRT
   <213> Artificial
<220>
   <223> Bioelastic polymer unit sequence
<400> 13
   Gly Val Gly Phe Pro
   1 5
<210> 14
   <211> 5
   <212> PRT
   <213> Artificial
<220>
   <223> Bioelastic polymer unit sequence
<400> 14
   Gly Phe Gly Phe Pro
   1 5
<210> 15
   <211> 5
   <212> PRT
   <213> Artificial
<220>
   <223> Bioelastic polymer unit sequence
<400> 15
   Gly Glu Gly Val Pro
    1              5
<210> 16
   <211> 5
   <212> PRT
   <213> Artificial
<220>
   <223> Bioelastic polymer unit sequence
<400> 16
   Gly Phe Gly Val Pro
    1              5
<210> 17
   <211> 5
   <212> PRT
   <213> Artificial
<220>
   <223> Bioelastic polymer unit sequence
<400> 17
   Gly Val Gly Ile Pro
    1              5
<210> 18
   <211> 800
   <212> PRT
   <213> Artificial
<220>
   <223> Thermosensitive bioelastic polymer sequence
<400> 18
   Val Pro Gly Val Gly Val Pro Gly Ala Gly Val Pro Gly Ala Gly Val
    1              5                   10                  15
   Pro Gly Ala Gly Val Pro Gly Ala Gly Val Pro Gly Ala Gly Val Pro
               20                  25                  30
   Gly Ala Gly Val Pro Gly Ala Gly Val Pro Gly Ala Gly Val Pro Gly
                   35                      40                      45
   Gly Gly Val Pro Gly Gly Gly Val Pro Gly Gly Gly Val Pro Gly Gly
           50                      55                      60
   Gly Val Pro Gly Gly Gly Val Pro Gly Gly Gly Val Pro Gly Gly Gly
   65                  70                      75                  80
   Val Pro Gly Val Gly Val Pro Gly Ala Gly Val Pro Gly Ala Gly Val
                   85                      90                      95
   Pro Gly Ala Gly Val Pro Gly Ala Gly Val Pro Gly Ala Gly Val Pro
               100                     105                     110
   Gly Ala Gly Val Pro Gly Ala Gly Val Pro Gly Ala Gly Val Pro Gly
           115                     120                     125
   Gly Gly Val Pro Gly Gly Gly Val Pro Gly Gly Gly Val Pro Gly Gly
       130                     135                     140
   Gly Val Pro Gly Gly Gly Val Pro Gly Gly Gly Val Pro Gly Gly Gly
   145                 150                     155                 160
   Val Pro Gly Val Gly Val Pro Gly Ala Gly Val Pro Gly Ala Gly Val
                   165                     170                     175
   Pro Gly Ala Gly Val Pro Gly Ala Gly Val Pro Gly Ala Gly Val Pro
               180                     185                     190
   Gly Ala Gly Val Pro Gly Ala Gly Val Pro Gly Ala Gly Val Pro Gly
           195                     200                     205
   Gly Gly Val Pro Gly Gly Gly Val Pro Gly Gly Gly Val Pro Gly Gly
       210                     215                     220
   Gly Val Pro Gly Gly Gly Val Pro Gly Gly Gly Val Pro Gly Gly Gly
   225                 230                     235                 240
   Val Pro Gly Val Gly Val Pro Gly Ala Gly Val Pro Gly Ala Gly Val
                   245                     250                     255
   Pro Gly Ala Gly Val Pro Gly Ala Gly Val Pro Gly Ala Gly Val Pro
               260                     265                     270
   Gly Ala Gly Val Pro Gly Ala Gly Val Pro Gly Ala Gly Val Pro Gly
           275                     280                     285
   Gly Gly Val Pro Gly Gly Gly Val Pro Gly Gly Gly Val Pro Gly Gly
       290                     295                     300
   Gly Val Pro Gly Gly Gly Val Pro Gly Gly Gly Val Pro Gly Gly Gly
   305                 310                     315                 320
   Val Pro Gly Val Gly Val Pro Gly Ala Gly Val Pro Gly Ala Gly Val
                   325                     330                     335
   Pro Gly Ala Gly Val Pro Gly Ala Gly Val Pro Gly Ala Gly Val Pro
               340                     345                     350
   Gly Ala Gly Val Pro Gly Ala Gly Val Pro Gly Ala Gly Val Pro Gly
           355                     360                     365
   Gly Gly Val Pro Gly Gly Gly Val Pro Gly Gly Gly Val Pro Gly Gly
       370                     375                     380
   Gly Val Pro Gly Gly Gly Val Pro Gly Gly Gly Val Pro Gly Gly Gly
   385                 390                     395                 400
   Val Pro Gly Val Gly Val Pro Gly Ala Gly Val Pro Gly Ala Gly Val
                   405                     410                     415
   Pro Gly Ala Gly Val Pro Gly Ala Gly Val Pro Gly Ala Gly Val Pro
               420                     425                     430
   Gly Ala Gly Val Pro Gly Ala Gly Val Pro Gly Ala Gly Val Pro Gly
           435                     440                     445
   Gly Gly Val Pro Gly Gly Gly Val Pro Gly Gly Gly Val Pro Gly Gly
       450                     455                     460
   Gly Val Pro Gly Gly Gly Val Pro Gly Gly Gly Val Pro Gly Gly Gly
   465                 470                     475                 480
   Val Pro Gly Val Gly Val Pro Gly Ala Gly Val Pro Gly Ala Gly Val
                   485                     490                     495
   Pro Gly Ala Gly Val Pro Gly Ala Gly Val Pro Gly Ala Gly Val Pro
               500                     505                     510
   Gly Ala Gly Val Pro Gly Ala Gly Val Pro Gly Ala Gly Val Pro Gly
           515                     520                     525
   Gly Gly Val Pro Gly Gly Gly Val Pro Gly Gly Gly Val Pro Gly Gly
       530                     535                     540
   Gly Val Pro Gly Gly Gly Val Pro Gly Gly Gly Val Pro Gly Gly Gly
   545                 550                     555                 560
   Val Pro Gly Val Gly Val Pro Gly Ala Gly Val Pro Gly Ala Gly Val
                   565                     570                     575
   Pro Gly Ala Gly Val Pro Gly Ala Gly Val Pro Gly Ala Gly Val Pro
                       580                     585                     590
   Gly Ala Gly Val Pro Gly Ala Gly Val Pro Gly Ala Gly Val Pro Gly
               595                     600                     605
   Gly Gly Val Pro Gly Gly Gly Val Pro Gly Gly Gly Val Pro Gly Gly
           610                     615                     620
   Gly Val Pro Gly Gly Gly Val Pro Gly Gly Gly Val Pro Gly Gly Gly
   625                     630                     635                     640
   Val Pro Gly Val Gly Val Pro Gly Ala Gly Val Pro Gly Ala Gly Val
                   645                     650                     655
   Pro Gly Ala Gly Val Pro Gly Ala Gly Val Pro Gly Ala Gly Val Pro
               660                     665                     670
   Gly Ala Gly Val Pro Gly Ala Gly Val Pro Gly Ala Gly Val Pro Gly
           675                     680                     685
   Gly Gly Val Pro Gly Gly Gly Val Pro Gly Gly Gly Val Pro Gly Gly
       690                     695                     700
   Gly Val Pro Gly Gly Gly Val Pro Gly Gly Gly Val Pro Gly Gly Gly
   705                     710                     715                     720
   Val Pro Gly Val Gly Val Pro Gly Ala Gly Val Pro Gly Ala Gly Val
                   725                     730                     735
   Pro Gly Ala Gly Val Pro Gly Ala Gly Val Pro Gly Ala Gly Val Pro
               740                     745                     750
   Gly Ala Gly Val Pro Gly Ala Gly Val Pro Gly Ala Gly Val Pro Gly
           755                     760                     765
   Gly Gly Val Pro Gly Gly Gly Val Pro Gly Gly Gly Val Pro Gly Gly
       770                     775                     780
   Gly Val Pro Gly Gly Gly Val Pro Gly Gly Gly Val Pro Gly Gly Gly
   785                     790                     795                     800
<210> 19
   <211> 560
   <212> PRT
   <213> Artificial
<220>
   <223> Bioelastic polymer sequence
<400> 19
   Val Pro Gly Val Gly Val Pro Gly Val Gly Val Pro Gly Val Gly Val
   1               5                   10                  15
   Pro Gly Val Gly Val Pro Gly Val Gly Val Pro Gly Val Gly Val Pro
               20                  25                  30
   Gly Lys Gly Val Pro Gly Val Gly Val Pro Gly Val Gly Val Pro Gly
           35                  40                  45
   Val Gly Val Pro Gly Val Gly Val Pro Gly Val Gly Val Pro Gly Val
       50                  55                  60
   Gly Val Pro Gly Lys Gly Val Pro Gly Val Gly Val Pro Gly Val Gly
   65                  70                  75                  80
   Val Pro Gly Val Gly Val Pro Gly Val Gly Val Pro Gly Val Gly Val
                   85                  90                  95
   Pro Gly Val Gly Val Pro Gly Lys Gly Val Pro Gly Val Gly Val Pro
               100                 105                 110
   Gly Val Gly Val Pro Gly Val Gly Val Pro Gly Val Gly Val Pro Gly
           115                 120                 125
   Val Gly Val Pro Gly Val Gly Val Pro Gly Lys Gly Val Pro Gly Val
       130                 135                 140
   Gly Val Pro Gly Val Gly Val Pro Gly Val Gly Val Pro Gly Val Gly
   145                 150                 155                 160
   Val Pro Gly Val Gly Val Pro Gly Val Gly Val Pro Gly Lys Gly Val
                   165                 170                 175
   Pro Gly Val Gly Val Pro Gly Val Gly Val Pro Gly Val Gly Val Pro
               180                 185                 190
   Gly Val Gly Val Pro Gly Val Gly Val Pro Gly Val Gly Val Pro Gly
           195                 200                 205
   Lys Gly Val Pro Gly Val Gly Val Pro Gly Val Gly Val Pro Gly Val
       210                 215                 220
   Gly Val Pro Gly Val Gly Val Pro Gly Val Gly Val Pro Gly Val Gly
   225                 230                 235                 240
   Val Pro Gly Lys Gly Val Pro Gly Val Gly Val Pro Gly Val Gly Val
                   245                 250                 255
   Pro Gly Val Gly Val Pro Gly Val Gly Val Pro Gly Val Gly Val Pro
               260                 265                 270
   Gly Val Gly Val Pro Gly Lys Gly Val Pro Gly Val Gly Val Pro Gly
           275                 280                 285
   Val Gly Val Pro Gly Val Gly Val Pro Gly Val Gly Val Pro Gly Val
       290                 295                 300
   Gly Val Pro Gly Val Gly Val Pro Gly Lys Gly Val Pro Gly Val Gly
   305                 310                 315                 320
   Val Pro Gly Val Gly Val Pro Gly Val Gly Val Pro Gly Val Gly Val
                   325                 330                 335
   Pro Gly Val Gly Val Pro Gly Val Gly Val Pro Gly Lys Gly Val Pro
               340                 345                 350
   Gly Val Gly Val Pro Gly Val Gly Val Pro Gly Val Gly Val Pro Gly
           355                 360                 365
   Val Gly Val Pro Gly Val Gly Val Pro Gly Val Gly Val Pro Gly Lys
       370                 375                 380
   Gly Val Pro Gly Val Gly Val Pro Gly Val Gly Val Pro Gly Val Gly
   385                 390                 395                 400
   Val Pro Gly Val Gly Val Pro Gly Val Gly Val Pro Gly Val Gly Val
                   405                 410                 415
   Pro Gly Lys Gly Val Pro Gly Val Gly Val Pro Gly Val Gly Val Pro
               420                 425                 430
   Gly Val Gly Val Pro Gly Val Gly Val Pro Gly Val Gly Val Pro Gly
           435                 440                 445
   Val Gly Val Pro Gly Lys Gly Val Pro Gly Val Gly Val Pro Gly Val
       450                 455                 460
   Gly Val Pro Gly Val Gly Val Pro Gly Val Gly Val Pro Gly Val Gly
   465                 470                 475                 480
   Val Pro Gly Val Gly Val Pro Gly Lys Gly Val Pro Gly Val Gly Val
                   485                 490                 495
   Pro Gly Val Gly Val Pro Gly Val Gly Val Pro Gly Val Gly Val Pro
               500                 505                 510
   Gly Val Gly Val Pro Gly Val Gly Val Pro Gly Lys Gly Val Pro Gly
           515                 520                 525
   Val Gly Val Pro Gly Val Gly Val Pro Gly Val Gly Val Pro Gly Val
       530                 535                 540
   Gly Val Pro Gly Val Gly Val Pro Gly Val Gly Val Pro Gly Lys Gly
   545                 550                 555                 560
<210> 20
   <211> 450
   <212> PRT
   <213> Artificial
<220>
   <223> Biolelastic polymer sequence
<400> 20
   Val Pro Gly Val Gly Val Pro Gly Val Gly Val Pro Gly Val Gly Val
   1 5 10 15
   Pro Gly Val Gly Val Pro Gly Val Gly Val Pro Gly Gly Gly Val Pro
   20 25 30
   Gly Gly Gly Val Pro Gly Gly Gly Val Pro Gly Ala Gly Val Pro Gly
   35 40 45
   Ala Gly Val Pro Gly Val Gly Val Pro Gly Val Gly Val Pro Gly Val
   50 55 60
   Gly Val Pro Gly Val Gly Val Pro Gly Val Gly Val Pro Gly Gly Gly
   65 70 75 80
   Val Pro Gly Gly Gly Val Pro Gly Gly Gly Val Pro Gly Ala Gly Val
   85 90 95
   Pro Gly Ala Gly Val Pro Gly Val Gly Val Pro Gly Val Gly Val Pro
   100 105 110
   Gly Val Gly Val Pro Gly Val Gly Val Pro Gly Val Gly Val Pro Gly
   115 120 125
   Gly Gly Val Pro Gly Gly Gly Val Pro Gly Gly Gly Val Pro Gly Ala
   130 135 140
   Gly Val Pro Gly Ala Gly Val Pro Gly Val Gly Val Pro Gly Val Gly
   145 150 155 160
   Val Pro Gly Val Gly Val Pro Gly Val Gly Val Pro Gly Val Gly Val
   165 170 175
   Pro Gly Gly Gly Val Pro Gly Gly Gly Val Pro Gly Gly Gly Val Pro
   180 185 190
   Gly Ala Gly Val Pro Gly Ala Gly Val Pro Gly Val Gly Val Pro Gly
   195 200 205
   Val Gly Val Pro Gly Val Gly Val Pro Gly Val Gly Val Pro Gly Val
   210 215 220
   Gly Val Pro Gly Gly Gly Val Pro Gly Gly Gly Val Pro Gly Gly Gly
   225 230 235 240
   Val Pro Gly Ala Gly Val Pro Gly Ala Gly Val Pro Gly Val Gly Val
   245 250 255
   Pro Gly Val Gly Val Pro Gly Val Gly Val Pro Gly Val Gly Val Pro
   260 265 270
   Gly Val Gly Val Pro Gly Gly Gly Val Pro Gly Gly Gly Val Pro Gly
   275 280 285
   Gly Gly Val Pro Gly Ala Gly Val Pro Gly Ala Gly Val Pro Gly Val
   290 295 300
   Gly Val Pro Gly Val Gly Val Pro Gly Val Gly Val Pro Gly Val Gly
   305 310 315 320
   Val Pro Gly Val Gly Val Pro Gly Gly Gly Val Pro Gly Gly Gly Val
   325 330 335
   Pro Gly Gly Gly Val Pro Gly Ala Gly Val Pro Gly Ala Gly Val Pro
   340 345 350
   Gly Val Gly Val Pro Gly Val Gly Val Pro Gly Val Gly Val Pro Gly
   355 360 365
   Val Gly Val Pro Gly Val Gly Val Pro Gly Gly Gly Val Pro Gly Gly
   370 375 380
   Gly Val Pro Gly Gly Gly Val Pro Gly Ala Gly Val Pro Gly Ala Gly
   385 390 395 400
   Val Pro Gly Val Gly Val Pro Gly Val Gly Val Pro Gly Val Gly Val
   405 410 415
   Pro Gly Val Gly Val Pro Gly Val Gly Val Pro Gly Gly Gly Val Pro
   420 425 430
   Gly Gly Gly Val Pro Gly Gly Gly Val Pro Gly Ala Gly Val Pro Gly
   435 440 445
   Ala Gly
   450
<210> 21
   <211> 150
   <212> PRT
   <213> Artificial
<220>
   <223> Bioelastic polymer sequence
<400> 21
   Val Pro Gly Val Gly Val Pro Gly Val Gly Val Pro Gly Val Gly Val
   1               5                   10                  15
   Pro Gly Val Gly Val Pro Gly Val Gly Val Pro Gly Val Gly Val Pro
               20                  25                  30
   Gly Val Gly Val Pro Gly Val Gly Val Pro Gly Val Gly Val Pro Gly
           35                  40                  45
   Val Gly Val Pro Gly Val Gly Val Pro Gly Val Gly Val Pro Gly Val
       50                  55                  60
   Gly Val Pro Gly Val Gly Val Pro Gly Val Gly Val Pro Gly Val Gly
   65                  70                  75                  80
   Val Pro Gly Val Gly Val Pro Gly Val Gly Val Pro Gly Val Gly Val
                   85                  90                  95
   Pro Gly Val Gly Val Pro Gly Val Gly Val Pro Gly Val Gly Val Pro
               100                 105                 110
   Gly Val Gly Val Pro Gly Val Gly Val Pro Gly Val Gly Val Pro Gly
           115                 120                 125
   Val Gly Val Pro Gly Val Gly Val Pro Gly Val Gly Val Pro Gly Val
       130                 135                 140
   Gly Val Pro Gly Val Gly
   145                 150
<210> 22
   <211> 945
   <212> DNA
   <213> Artificial
<220>
   <223> Elastin-like polypeptide coding sequence
<220>
   <221> CDS
   <222> (1)..(942)
<400> 22
   atg agc ggg ccg ggc gtg ggt gtt ccg ggc gta ggt gtc cca ggt gtg      48
   Met Ser Gly Pro Gly Val Gly Val Pro Gly Val Gly Val Pro Gly Val
   1               5                   10                  15
   ggc gta ccg ggc gtt ggt gtt cct ggt gtc ggc gtg ccg ggc gtg ggt      96
   Gly Val Pro Gly Val Gly Val Pro Gly Val Gly Val Pro Gly Val Gly
               20                  25                  30
   gtt ccg ggc gta ggt gtc cca ggt gtg ggc gta ccg ggc gtt ggt gtt      144
   Val Pro Gly Val Gly Val Pro Gly Val Gly Val Pro Gly Val Gly Val
           35                  40                  45
   cct ggt gtc ggc gtg ccg ggc gtg ggt gtt ccg ggc gta ggt gtc cca      192
   Pro Gly Val Gly Val Pro Gly Val Gly Val Pro Gly Val Gly Val Pro
       50                  55                  60
   ggt gtg ggc gta ccg ggc gtt ggt gtt cct ggt gtc ggc gtg ccg ggc      240
   Gly Val Gly Val Pro Gly Val Gly Val Pro Gly Val Gly Val Pro Gly
   65                  70                  75                  80
   gtg ggt gtt ccg ggc gta ggt gtc cca ggt gtg ggc gta ccg ggc gtt      288
   Val Gly Val Pro Gly Val Gly Val Pro Gly Val Gly Val Pro Gly Val
                   85                  90                  95
   ggt gtt cct ggt gtc ggc gtg ccg ggc gtg ggt gtt ccg ggc gta ggt      336
   Gly Val Pro Gly Val Gly Val Pro Gly Val Gly Val Pro Gly Val Gly
               100                 105                 110
   gtc cca ggt gtg ggc gta ccg ggc gtt ggt gtt cct ggt gtc ggc gtg      384
   Val Pro Gly Val Gly Val Pro Gly Val Gly Val Pro Gly Val Gly Val
           115                 120                 125
   ccg ggc gtg ggt gtt ccg ggc gta ggt gtc cca ggt gtg ggc gta ccg      432
   Pro Gly Val Gly Val Pro Gly Val Gly Val Pro Gly Val Gly Val Pro
       130                 135                 140
   ggc gtt ggt gtt cct ggt gtc ggc gtg ccg ggc ggg ccg ggc ggc agc      480
   Gly Val Gly Val Pro Gly Val Gly Val Pro Gly Gly Pro Gly Gly Ser
   145                 150                 155                 160
   ggc ggc agc gga tcc ggg aga aaa tcc agc aag atg caa gcc ttc aga      528
   Gly Gly Ser Gly Ser Gly Arg Lys Ser Ser Lys Met Gln Ala Phe Arg
                   165                 170                 175
   atc tgg gat gtt aac cag aag acc ttc tat ctg agg aac aac caa cta      576
   Ile Trp Asp Val Asn Gln Lys Thr Phe Tyr Leu Arg Asn Asn Gln Leu
               180                 185                 190
   gtt gct gga tac ttg caa gga cca aat gtc aat tta gaa gaa aag ata      624
   Val Ala Gly Tyr Leu Gln Gly Pro Asn Val Asn Leu Glu Glu Lys Ile
           195                 200                 205
   gat gtg gta ccc att gag cct cat gct ctg ttc ttg gga atc cat gga      672
   Asp Val Val Pro Ile Glu Pro His Ala Leu Phe Leu Gly Ile His Gly
       210                 215                 220
   ggg aag atg tgc ctg tcc tgt gtc aag tct ggt gat gag acc aga ctc      720
   Gly Lys Met Cys Leu Ser Cys Val Lys Ser Gly Asp Glu Thr Arg Leu
   225                 230                 235                 240
   cag ctg gag gca gtt aac atc act gac ctg agc gag aac aga aag cag      768
   Gln Leu Glu Ala Val Asn Ile Thr Asp Leu Ser Glu Asn Arg Lys Gln
                   245                 250                 255
   gac aag cgc ttc gcc ttc atc cgc tca gac agc ggc ccc acc acc agt      816
   Asp Lys Arg Phe Ala Phe Ile Arg Ser Asp Ser Gly Pro Thr Thr Ser
               260                 265                 270
   ttt gag tct gcc gcc tgc ccc ggt tgg ttc ctc tgc aca gcg atg gaa      864
   Phe Glu Ser Ala Ala Cys Pro Gly Trp Phe Leu Cys Thr Ala Met Glu
           275                 280                 285
   gct gac cag ccc gtc agc ctc acc aat atg cct gac gaa ggc gtc atg      912
   Ala Asp Gln Pro Val Ser Leu Thr Asn Met Pro Asp Glu Gly Val Met
       290                 295                 300
   gtc acc aaa ttc tac ttc cag gag gac gag tag                          945
   Val Thr Lys Phe Tyr Phe Gln Glu Asp Glu
   305                 310
<210> 23
   <211> 314
   <212> PRT
   <213> Artificial
<220>
   <223> Synthetic Construct
<400> 23
   Met Ser Gly Pro Gly Val Gly Val Pro Gly Val Gly Val Pro Gly Val
   1               5                   10                  15
   Gly Val Pro Gly Val Gly Val Pro Gly Val Gly Val Pro Gly Val Gly
               20                  25                  30
   Val Pro Gly Val Gly Val Pro Gly Val Gly Val Pro Gly Val Gly Val
           35                  40                  45
   Pro Gly Val Gly Val Pro Gly Val Gly Val Pro Gly Val Gly Val Pro
       50                  55                  60
   Gly Val Gly Val Pro Gly Val Gly Val Pro Gly Val Gly Val Pro Gly
   65                  70                  75                  80
   Val Gly Val Pro Gly Val Gly Val Pro Gly Val Gly Val Pro Gly Val
                   85                  90                  95
   Gly Val Pro Gly Val Gly Val Pro Gly Val Gly Val Pro Gly Val Gly
               100                 105                 110
   Val Pro Gly Val Gly Val Pro Gly Val Gly Val Pro Gly Val Gly Val
           115                 120                 125
   Pro Gly Val Gly Val Pro Gly Val Gly Val Pro Gly Val Gly Val Pro
       130                 135                 140
   Gly Val Gly Val Pro Gly Val Gly Val Pro Gly Gly Pro Gly Gly Ser
   145                 150                 155                 160
   Gly Gly Ser Gly Ser Gly Arg Lys Ser Ser Lys Met Gln Ala Phe Arg
                   165                 170                 175
   Ile Trp Asp Val Asn Gln Lys Thr Phe Tyr Leu Arg Asn Asn Gln Leu
               180                 185                 190
   Val Ala Gly Tyr Leu Gln Gly Pro Asn Val Asn Leu Glu Glu Lys Ile
           195                 200                 205
   Asp Val Val Pro Ile Glu Pro His Ala Leu Phe Leu Gly Ile His Gly
       210                 215                 220
   Gly Lys Met Cys Leu Ser Cys Val Lys Ser Gly Asp Glu Thr Arg Leu
   225                 230                 235                 240
   Gln Leu Glu Ala Val Asn Ile Thr Asp Leu Ser Glu Asn Arg Lys Gln
                   245                 250                 255
   Asp Lys Arg Phe Ala Phe Ile Arg Ser Asp Ser Gly Pro Thr Thr Ser
               260                 265                 270
   Phe Glu Ser Ala Ala Cys Pro Gly Trp Phe Leu Cys Thr Ala Met Glu
           275                 280                 285
   Ala Asp Gln Pro Val Ser Leu Thr Asn Met Pro Asp Glu Gly Val Met
       290                 295                 300
   Val Thr Lys Phe Tyr Phe Gln Glu Asp Glu
   305                 310
<210> 24
   <211> 1845
   <212> DNA
   <213> Artificial
<220>
   <223> Elastin-like polypeptide coding sequence
<220>
   <221> CDS
   <222> (1)..(1842)
<400> 24
   atg agc ggg ccg ggc gtg ggt gtt ccg ggc gtg ggt gtt ccg ggt ggc       48
   Met Ser Gly Pro Gly Val Gly Val Pro Gly Val Gly Val Pro Gly Gly
   1               5                   10                  15
   ggt gtg ccg ggc gca ggt gtt cct ggt gta ggt gtg ccg ggt gtt ggt       96
   Gly Val Pro Gly Ala Gly Val Pro Gly Val Gly Val Pro Gly Val Gly
               20                  25                  30
   gtg ccg ggt gtt ggt gta cca ggt ggc ggt gtt ccg ggt gca ggc gtt      144
   Val Pro Gly Val Gly Val Pro Gly Gly Gly Val Pro Gly Ala Gly Val
           35                  40                  45
   ccg ggt ggc ggt gtg ccg ggc gtg ggt gtt ccg ggc gtg ggt gtt ccg      192
   Pro Gly Gly Gly Val Pro Gly Val Gly Val Pro Gly Val Gly Val Pro
       50                  55                  60
   ggt ggc ggt gtg ccg ggc gca ggt gtt cct ggt gta ggt gtg ccg ggt      240
   Gly Gly Gly Val Pro Gly Ala Gly Val Pro Gly Val Gly Val Pro Gly
   65                  70                  75                  80
   gtt ggt gtg ccg ggt gtt ggt gta cca ggt ggc ggt gtt ccg ggt gca      288
   Val Gly Val Pro Gly Val Gly Val Pro Gly Gly Gly Val Pro Gly Ala
                   85                  90                  95
   ggc gtt ccg ggt ggc ggt gtg ccg ggc gtg ggt gtt ccg ggc gtg ggt      336
   Gly Val Pro Gly Gly Gly Val Pro Gly Val Gly Val Pro Gly Val Gly
               100                 105                 110
   gtt ccg ggt ggc ggt gtg ccg ggc gca ggt gtt cct ggt gta ggt gtg      384
   Val Pro Gly Gly Gly Val Pro Gly Ala Gly Val Pro Gly Val Gly Val
           115                 120                 125
   ccg ggt gtt ggt gtg ccg ggt gtt ggt gta cca ggt ggc ggt gtt ccg      432
   Pro Gly Val Gly Val Pro Gly Val Gly Val Pro Gly Gly Gly Val Pro
       130                 135                 140
   ggt gca ggc gtt ccg ggt ggc ggt gtg ccg ggc gtg ggt gtt ccg ggc      480
   Gly Ala Gly Val Pro Gly Gly Gly Val Pro Gly Val Gly Val Pro Gly
   145                 150                 155                 160
   gtg ggt gtt ccg ggt ggc ggt gtg ccg ggc gca ggt gtt cct ggt gta      528
   Val Gly Val Pro Gly Gly Gly Val Pro Gly Ala Gly Val Pro Gly Val
                   165                 170                 175
   ggt gtg ccg ggt gtt ggt gtg ccg ggt gtt ggt gta cca ggt ggc ggt      576
   Gly Val Pro Gly Val Gly Val Pro Gly Val Gly Val Pro Gly Gly Gly
               180                 185                 190
   gtt ccg ggt gca ggc gtt ccg ggt ggc ggt gtg ccg ggc gtg ggt gtt      624
   Val Pro Gly Ala Gly Val Pro Gly Gly Gly Val Pro Gly Val Gly Val
           195                 200                 205
   ccg ggc gtg ggt gtt ccg ggt ggc ggt gtg ccg ggc gca ggt gtt cct      672
   Pro Gly Val Gly Val Pro Gly Gly Gly Val Pro Gly Ala Gly Val Pro
       210                 215                 220
   ggt gta ggt gtg ccg ggt gtt ggt gtg ccg ggt gtt ggt gta cca ggt      720
   Gly Val Gly Val Pro Gly Val Gly Val Pro Gly Val Gly Val Pro Gly
   225                 230                 235                 240
   ggc ggt gtt ccg ggt gca ggc gtt ccg ggt ggc ggt gtg ccg ggc gtg      768
   Gly Gly Val Pro Gly Ala Gly Val Pro Gly Gly Gly Val Pro Gly Val
                   245                 250                 255
   ggt gtt ccg ggc gtg ggt gtt ccg ggt ggc ggt gtg ccg ggc gca ggt      816
   Gly Val Pro Gly Val Gly Val Pro Gly Gly Gly Val Pro Gly Ala Gly
               260                 265                 270
   gtt cct ggt gta ggt gtg ccg ggt gtt ggt gtg ccg ggt gtt ggt gta      864
   Val Pro Gly Val Gly Val Pro Gly Val Gly Val Pro Gly Val Gly Val
           275                 280                 285
   cca ggt ggc ggt gtt ccg ggt gca ggc gtt ccg ggt ggc ggt gtg ccg      912
   Pro Gly Gly Gly Val Pro Gly Ala Gly Val Pro Gly Gly Gly Val Pro
       290                 295                 300
   ggc gtg ggt gtt ccg ggc gtg ggt gtt ccg ggt ggc ggt gtg ccg ggc      960
   Gly Val Gly Val Pro Gly Val Gly Val Pro Gly Gly Gly Val Pro Gly
   305                     310                     315                     320
   gca ggt gtt cct ggt gta ggt gtg ccg ggt gtt ggt gtg ccg ggt gtt          1008
   Ala Gly Val Pro Gly Val Gly Val Pro Gly Val Gly Val Pro Gly Val
                           325                     330                     335
   ggt gta cca ggt ggc ggt gtt ccg ggt gca ggc gtt ccg ggt ggc ggt          1056
   Gly Val Pro Gly Gly Gly Val Pro Gly Ala Gly Val Pro Gly Gly Gly
                   340                     345                     350
   gtg ccg ggc gtg ggt gtt ccg ggc gtg ggt gtt ccg ggt ggc ggt gtg          1104
   Val Pro Gly Val Gly Val Pro Gly Val Gly Val Pro Gly Gly Gly Val
           355                     360                     365
   ccg ggc gca ggt gtt cct ggt gta ggt gtg ccg ggt gtt ggt gtg ccg          1152
   Pro Gly Ala Gly Val Pro Gly Val Gly Val Pro Gly Val Gly Val Pro
       370                     375                     380
   ggt gtt ggt gta cca ggt ggc ggt gtt ccg ggt gca ggc gtt ccg ggt          1200
   Gly Val Gly Val Pro Gly Gly Gly Val Pro Gly Ala Gly Val Pro Gly
   385                     390                     395                     400
   ggc ggt gtg ccg ggc gtg ggt gtt ccg ggc gtg ggt gtt ccg ggt ggc          1248
   Gly Gly Val Pro Gly Val Gly Val Pro Gly Val Gly Val Pro Gly Gly
                           405                     410                     415
   ggt gtg ccg ggc gca ggt gtt cct ggt gta ggt gtg ccg ggt gtt ggt          1296
   Gly Val Pro Gly Ala Gly Val Pro Gly Val Gly Val Pro Gly Val Gly
                   420                     425                     430
   gtg ccg ggt gtt ggt gta cca ggt ggc ggt gtt ccg ggt gca ggc gtt          1344
   Val Pro Gly Val Gly Val Pro Gly Gly Gly Val Pro Gly Ala Gly Val
           435                     440                     445
   ccg ggt ggc ggt gtg ccg ggc ggg ccg ggc ggc agc ggc ggc agc gga          1392
   Pro Gly Gly Gly Val Pro Gly Gly Pro Gly Gly Ser Gly Gly Ser Gly
       450                     455                     460
   tcc ggg aga aaa tcc agc aag atg caa gcc ttc aga atc tgg gat gtt          1440
   Ser Gly Arg Lys Ser Ser Lys Met Gln Ala Phe Arg Ile Trp Asp Val
   465                     470                     475                     480
   aac cag aag acc ttc tat ctg agg aac aac caa cta gtt gct gga tac          1488
   Asn Gln Lys Thr Phe Tyr Leu Arg Asn Asn Gln Leu Val Ala Gly Tyr
                           485                     490                     495
   ttg caa gga cca aat gtc aat tta gaa gaa aag ata gat gtg gta ccc          1536
   Leu Gln Gly Pro Asn Val Asn Leu Glu Glu Lys Ile Asp Val Val Pro
                   500                     505                     510
   att gag cct cat gct ctg ttc ttg gga atc cat gga ggg aag atg tgc          1584
   Ile Glu Pro His Ala Leu Phe Leu Gly Ile His Gly Gly Lys Met Cys
           515                     520                     525
   ctg tcc tgt gtc aag tct ggt gat gag acc aga ctc cag ctg gag gca          1632
   Leu Ser Cys Val Lys Ser Gly Asp Glu Thr Arg Leu Gln Leu Glu Ala
       530                     535                     540
   gtt aac atc act gac ctg agc gag aac aga aag cag gac aag cgc ttc          1680
   Val Asn Ile Thr Asp Leu Ser Glu Asn Arg Lys Gln Asp Lys Arg Phe
   545                     550                     555                     560
   gcc ttc atc cgc tca gac agc ggc ccc acc acc agt ttt gag tct gcc          1728
   Ala Phe Ile Arg Ser Asp Ser Gly Pro Thr Thr Ser Phe Glu Ser Ala
                           565                     570                     575
   gcc tgc ccc ggt tgg ttc ctc tgc aca gcg atg gaa gct gac cag ccc          1776
   Ala Cys Pro Gly Trp Phe Leu Cys Thr Ala Met Glu Ala Asp Gln Pro
                   580                     585                     590
   gtc agc ctc acc aat atg cct gac gaa ggc gtc atg gtc acc aaa ttc          1824
   Val Ser Leu Thr Asn Met Pro Asp Glu Gly Val Met Val Thr Lys Phe
           595                     600                     605
   tac ttc cag gag gac gag tag                                              1845
   Tyr Phe Gln Glu Asp Glu
       610
<210> 25
   <211> 614
   <212> PRT
   <213> Artificial
<220>
   <223> Synthetic Construct
<400> 25
   Met Ser Gly Pro Gly Val Gly Val Pro Gly Val Gly Val Pro Gly Gly
   1 5 10 15
   Gly Val Pro Gly Ala Gly Val Pro Gly Val Gly Val Pro Gly Val Gly
   20 25 30
   Val Pro Gly Val Gly Val Pro Gly Gly Gly Val Pro Gly Ala Gly Val
   35 40 45
   Pro Gly Gly Gly Val Pro Gly Val Gly Val Pro Gly Val Gly Val Pro
   50 55 60
   Gly Gly Gly Val Pro Gly Ala Gly Val Pro Gly Val Gly Val Pro Gly
   65 70 75 80
   Val Gly Val Pro Gly Val Gly Val Pro Gly Gly Gly Val Pro Gly Ala
   85 90 95
   Gly Val Pro Gly Gly Gly Val Pro Gly Val Gly Val Pro Gly Val Gly
   100 105 110
   Val Pro Gly Gly Gly Val Pro Gly Ala Gly Val Pro Gly Val Gly Val
   115 120 125
   Pro Gly Val Gly Val Pro Gly Val Gly Val Pro Gly Gly Gly Val Pro
   130 135 140
   Gly Ala Gly Val Pro Gly Gly Gly Val Pro Gly Val Gly Val Pro Gly
   145 150 155 160
   Val Gly Val Pro Gly Gly Gly Val Pro Gly Ala Gly Val Pro Gly Val
   165 170 175
   Gly Val Pro Gly Val Gly Val Pro Gly Val Gly Val Pro Gly Gly Gly
   180 185 190
   Val Pro Gly Ala Gly Val Pro Gly Gly Gly Val Pro Gly Val Gly Val
   195 200 205
   Pro Gly Val Gly Val Pro Gly Gly Gly Val Pro Gly Ala Gly Val Pro
   210 215 220
   Gly Val Gly Val Pro Gly Val Gly Val Pro Gly Val Gly Val Pro Gly
   225 230 235 240
   Gly Gly Val Pro Gly Ala Gly Val Pro Gly Gly Gly Val Pro Gly Val
   245 250 255
   Gly Val Pro Gly Val Gly Val Pro Gly Gly Gly Val Pro Gly Ala Gly
   260 265 270
   Val Pro Gly Val Gly Val Pro Gly Val Gly Val Pro Gly Val Gly Val
   275 280 285
   Pro Gly Gly Gly Val Pro Gly Ala Gly Val Pro Gly Gly Gly Val Pro
   290 295 300
   Gly Val Gly Val Pro Gly Val Gly Val Pro Gly Gly Gly Val Pro Gly
   305 310 315 320
   Ala Gly Val Pro Gly Val Gly Val Pro Gly Val Gly Val Pro Gly Val
   325 330 335
   Gly Val Pro Gly Gly Gly Val Pro Gly Ala Gly Val Pro Gly Gly Gly
   340 345 350
   Val Pro Gly Val Gly Val Pro Gly Val Gly Val Pro Gly Gly Gly Val
   355 360 365
   Pro Gly Ala Gly Val Pro Gly Val Gly Val Pro Gly Val Gly Val Pro
   370 375 380
   Gly Val Gly Val Pro Gly Gly Gly Val Pro Gly Ala Gly Val Pro Gly
   385 390 395 400
   Gly Gly Val Pro Gly Val Gly Val Pro Gly Val Gly Val Pro Gly Gly
   405 410 415
   Gly Val Pro Gly Ala Gly Val Pro Gly Val Gly Val Pro Gly Val Gly
   420 425 430
   Val Pro Gly Val Gly Val Pro Gly Gly Gly Val Pro Gly Ala Gly Val
   435 440 445
   Pro Gly Gly Gly Val Pro Gly Gly Pro Gly Gly Ser Gly Gly Ser Gly
   450 455 460
   Ser Gly Arg Lys Ser Ser Lys Met Gln Ala Phe Arg Ile Trp Asp Val
   465 470 475 480
   Asn Gln Lys Thr Phe Tyr Leu Arg Asn Asn Gln Leu Val Ala Gly Tyr
   485 490 495
   Leu Gln Gly Pro Asn Val Asn Leu Glu Glu Lys Ile Asp Val Val Pro
   500 505 510
   Ile Glu Pro His Ala Leu Phe Leu Gly Ile His Gly Gly Lys Met Cys
   515 520 525
   Leu Ser Cys Val Lys Ser Gly Asp Glu Thr Arg Leu Gln Leu Glu Ala
       530                      535                     540
   Val Asn Ile Thr Asp Leu Ser Glu Asn Arg Lys Gln Asp Lys Arg Phe
   545                 550                     555                 560
   Ala Phe Ile Arg Ser Asp Ser Gly Pro Thr Thr Ser Phe Glu Ser Ala
                   565                     570                 575
   Ala Cys Pro Gly Trp Phe Leu Cys Thr Ala Met Glu Ala Asp Gln Pro
               580                     585                 590
   Val Ser Leu Thr Asn Met Pro Asp Glu Gly Val Met Val Thr Lys Phe
           595                 600                     605
   Tyr Phe Gln Glu Asp Glu
       610

## Description

This invention was made with Government support under Grant Nos. AR047442 and EB002263 from the National Institutes of Health. The Government has certain rights to this invention.

### Related Applications

This application claims the benefit of United States Provisional Patent Application Serial No. 60/693,966, filed June 24, 2005.

### Field of the Invention

The present invention concerns compositions for the controlled released delivery of pharmaceutical compounds.

### Background of the Invention

Osteoarthritis (OA) is a degenerative joint disease that affects more patients than any other musculoskeletal disorder. It accounts for over 4 million hospitalizations every year and its prevalence is estimated to increase by over 50% in the next 20 years. OA affects individual joints and was historically considered to arise from a "wear and tear" due to joint loading. OA is now understood to arise from multiple causes, including biochemical, genetic and biomechanical factors, that interact and promote the progression of joint disease. While surgical options for joint re-alignment (osteotomy), prosthetic replacement (arthroplasty), or fusion (arthrodesis) are available to patients with advanced joint degeneration, only a small fraction of patients need operative treatments. Hence, there is a great and increasing need to provide non-surgical treatments that are capable of both alleviating patient symptoms and modifying the course of disease progression.

The most widely available treatments for the patient with early to moderate joint disease are the chronic use of NSAIDs. Although these drugs provide effectiveness in pain relief, they are associated with frequent adverse conditions including gastrointestinal bleeding and ulceration. Additionally, NSAIDs are reported to inhibit cell biosynthesis which may compromise the long-term intrinsic repair process in joint degeneration. More recently protein drugs with the ability to modify the disease state of OA have been identified. These protein drugs include TNF-a inhibitors and IL-1 inhibitors and have shown significant promise both in preclinical and clinical studies in preventing the onset and retarding the progression of the disease.

Since OA is a disease that is localized to a few joints at a time, localized delivery of the therapeutic is highly preferred. One such technique is the administration of the drug directly into the affected joint cavity, i.e. intra-articular injection, which is currently recommended for corticosteroids and hyaluronan solutions in treating OA. Although the intra-articular mechanism of drug delivery is attractive to the patient and clinician alike, it is compromised by the presence of a highly efficient lymphatic system that rapidly clears molecules from the synovial cavity. Consequently, the therapeutic drug has to be administered frequently or at high concentrations to be effective. This, in turn, may be costly and result in adverse side effects and high levels of patient discomfort. Controlled drug delivery systems have been sought-after to overcome these challenges.

US Patent No. 6,328,996 to Urry describes bioelastomeric drug delivery systems, but does not suggest them for administration directly into a region of interest.

WO 00/56774 A1 discloses a composition comprising a fusion protein comprising a protein in combination with an elastin-like protein (ELP) containing the repeat (VPGXG)n, wherein X is any amino acid, such as Val, Ala or Gly, with X being the same or different in each repeat unit. A fusion protein comprising the protein thioredoxin and an ELP comprising [(VPGVG)5(VPGAG)2(VPGGG)3]9 is specifically disclosed. The fusion protein is prepared for the targeted delivery of proteins to solid tumors by hyperthermia after systemic administration.

### Summary of the Invention

The scope of the invention is defined by the claims. Any references in the description to methods of treatment refer to the compounds, pharmaceutical compositions and medicaments of the present invention for use in a method for treatment of the human (or animal) body by therapy (or for diagnosis).

An objective of the present invention is to provide a thermally responsive biocompatible polymer for the sustained delivery of drugs such as IL-1 receptor antagonist. The delivery system in the present invention utilizes the unique rapid aggregation and slow disaggregation properties of thermally responsive biopolymers to deliver protein drugs directly to a pathologic site, such as an OA affected joint.

Herein provided is a method for delivering a drug depot of a compound of interest to a selected region in a subject. The method comprises administering a composition directly to said region of interest, the composition comprising said compound to be delivered and a polymer that undergoes an inverse temperature phase transition, so that a sustained release of said compound of interest at said selected region is provided.

In some embodiments a method is provided for delivering a drug depot of a compound of interest to a selected region in a subject (e.g., a joint or synovial joint). The method comprises administering a composition to region of interest (e.g., directly, such as by injection in or to the region of interest). The composition comprises the compound to be delivered and a polymer that undergoes an inverse temperature phase transition. The polymer has a transition temperature (Tₜ) less than the body temperature of the subject (e.g., less than 37 °C). The composition or conjugate aggregates in the region of interest and then gradually disaggregates, providing a sustained or controlled release of the compound of interest at the selected region.

In some embodiments a method is provided for delivering a drug depot of a compound of interest to a selected region in a subject, said method comprising: administering a composition directly to said region of interest, said composition comprising said compound to be delivered and a polymer that undergoes an inverse temperature phase transition; wherein said polymer has a transition temperature (Tₜ) less than the body temperature of said subject; so that said composition (or said conjugate) separates from solution in said region of interest to form a bulk aggregate, and then gradually separates from bulk aggregate to go back to solution, providing a sustained release of said compound of interest at said selected region (or stated differently, so that said conjugate separates from solution in said region of interest and then gradually goes back to solution phase, providing a sustained release of said compound of interest at said selected region.).

In some embodiments the composition comprises the compound to be delivered conjugated to the polymer; in other embodiments the composition comprises the compound to be delivered mixed with, but not otherwise conjugated to or chemically coupled to, the polymer.

The present invention concerns a pharmaceutically acceptable composition for delivering a drug depot by injection to a selected region in a subject, wherein said composition comprises a therapeutic compound coupled to a polymer as a fusion protein, wherein said polymer undergoes an inverse temperature phase transition and has a transition temperature less than the body temperature of the subject such that a sustained release of said therapeutic compound is provided, and further wherein said polymer is an Elastin-like polypeptide (ELP) comprising [(VPGVG)5(VPGGG)3(VPGAG)2]9 (SEQ. ID. NO:20).

A further aspect of the invention is a pharmaceutically acceptable composition comprising a therapeutic compound coupled to a polymer as a fusion protein, wherein said polymer undergoes an inverse temperature phase transition and has a transition temperature less than the body temperature of a subject to be treated such that a sustained release of said therapeutic compound is provided upon injection, and further wherein said polymer is an Elastin-like polypeptide (ELP) comprising [(VPGVG)5(VPGGG)3(VPGAG)2]9 (SEQ. ID. NO:20), for use as a medicament.

Examples of such therapeutic compounds include but are not limited to TNF antibodies, IL-1 antibodies, soluble TNF receptors, soluble IL-1 receptors, TNF receptor antagonists, and IL-1 receptor antagonists, with a particular example being recombinant human IL-1 receptor antagonist and its isoforms.

A further aspect of the invention is an injection device (including syringes and other injection devices) containing the composition as claimed.

### Brief Description of the Drawings

In the following figures, only the compositions comprising [(VPGVG)5(VPGGG)3(VPGAG)2]9 (SEQ. ID. NO:20) are according to the invention. Compositions comprising other polymers are disclosed for reference purposes.

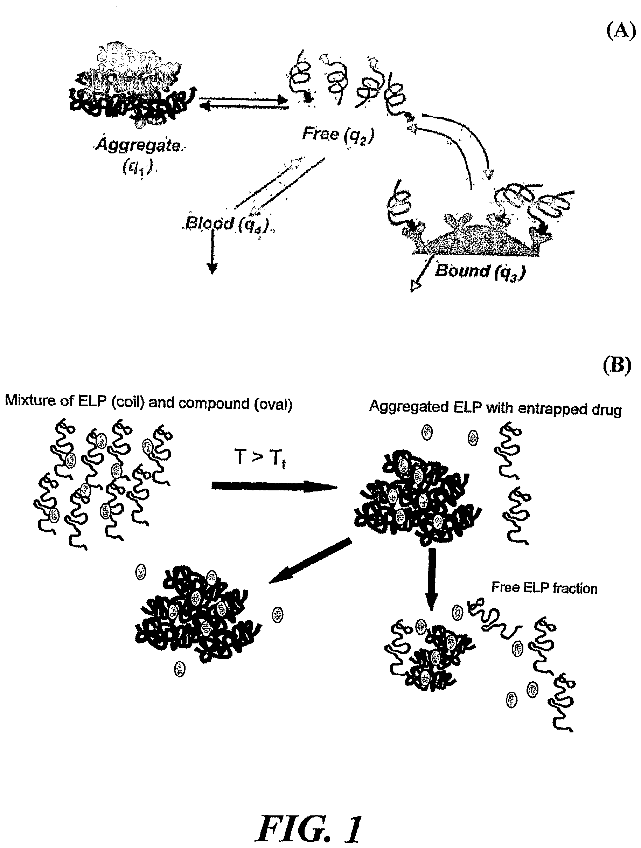

**Figure 1**. Schematic illustrating examples of a proposed drug delivery system, (a) A compound (drug) attached to a thermosensitive bioelastic polymer (ELP) may be injected into a tissue region (e.g., joint cavity), and the release of the compound occurs over time from an aggregate. In its free form, the compound is available to bind to a receptor as shown. The "free" fraction of compound-bioelastic polymer will be cleared from the tissue region (e.g., joint cavity) over time. (b) Alternate example showing a compound initially mixed with a thermosensitive bioelastic polymer during or prior to delivery to a tissue region, promoting entrapment of the compound. The release of the compound occurs over time, with the "free" compound cleared from the tissue region over time.

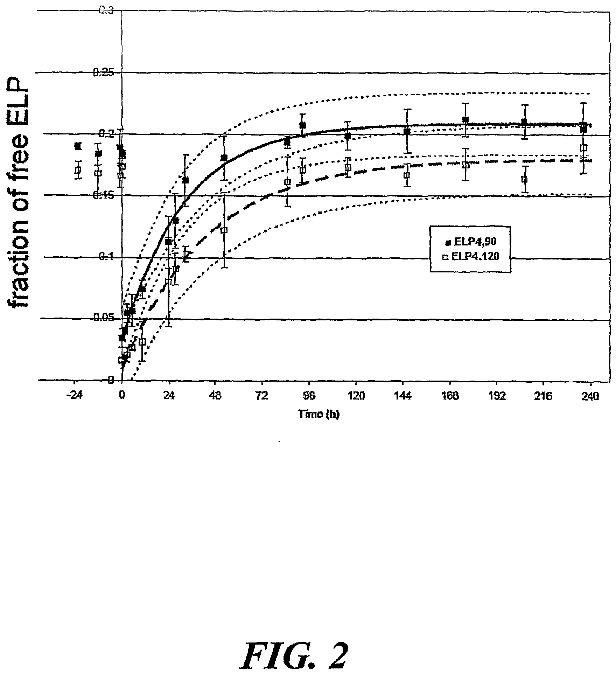

**Figure 2****.** Fraction of ELP detected in supernatant over time, following equilibration of an ELP aggregate at 37°C. Results are shown for ELP4-90 ([(VPGVG)₁₀]₉, **SEQ ID NO:1**) and ELP4-120 ([(VPGVG)₁₀]₁₂, **SEQ ID NO:2**) (mean ± SD, n=4). ELP above its transition temperature was equilibrated at 37°C to promote the inverse temperature phase transition. Supernatant was replaced with fresh PBS at 37°C and ELP concentration in aliquots of supernatant was quantified over time by absorbance at 280 nm. Data were fit to a first-order exponential function (solid line) of the form [ELP]=[ELP]ₛₛ - A exp(-t/τ), to obtain a time constant of equilibration (τ) and a fraction of ELP not contained in aggregate form at equilibrium ([ELP]ₛₛ). For ELP4-90, constants were found to be: [ELP]ₛₛ = 0.21 and τ= 31h. For ELP4-120, constants were found to be: [ELP]ₛₛ = 0.18 and τ= 43h.

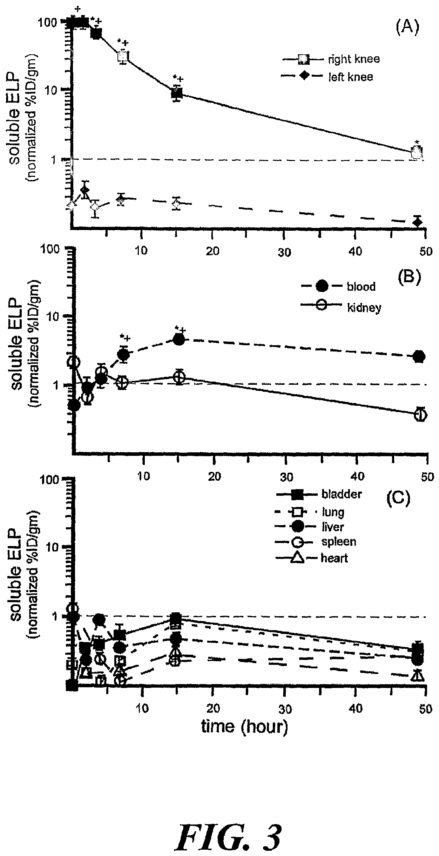

**Figure 3****.** Biodistribution of non-aggregating [¹⁴C]ELP (Tₜ > 50°C) after intra-articular injection into the right knee joint in a rat model. The injected dose (ID/gm) : is the amount of ¹⁴C recovered from the injected knee compartment (per gram of recovered tissues and fluids) at 10 min post injection. For individual compartments, ¹⁴C (per gram of recovered tissue or fluid) was normalized by this value to determine a %ID/gm for each tissue or fluid. All data expressed as mean ± SE (n=5). (A) * p<0.05, statistically different from time zero; + p<0.05, statistically different from uninjected (left) knee compartment. (B) * p<0.05, statistically different from time zero; + p<0.05, statistically different from blood. (C) The concentration of [¹⁴C]ELP was found to be below 1 %ID/gm for all organs, and these tissues were not found to be statistically different from the uninjected (left) knee at all time points.

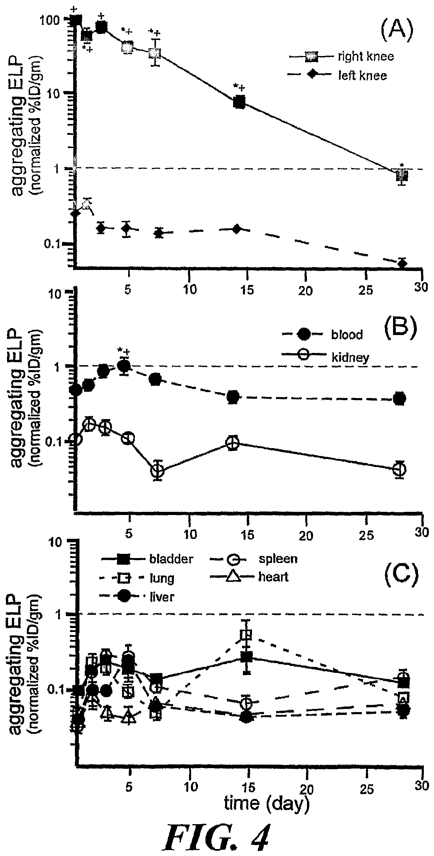

**Figure 4****.** Biodistribution of the aggregating [¹⁴C]ELP (Tₜ < 35°C) after intra-articular injection into the right knee joint of a rat model. The injected dose (ID) is the amount of ¹⁴C recovered from the injected knee compartment (per gram of recovered tissues and fluids) at time zero (10 min post injection). For individual compartments, ¹⁴C (per gram of recovered tissue or fluid) was normalized by this value to determine a %ID/gm for each tissue or fluid. All data expressed as mean ± SE (n=5). (A) * p<0.05, statistically different from time zero; + p<0.05, statistically different from uninjected (left) knee compartment. (B) * p<0.05, statistically different from time zero; + p<0.05, statistically different from blood. (C) The concentration of [¹⁴C]ELP was found to be below 1 %ID/gm for all organs, and these tissues were not found to be statistically different from the uninjected (left) knee at all time points.

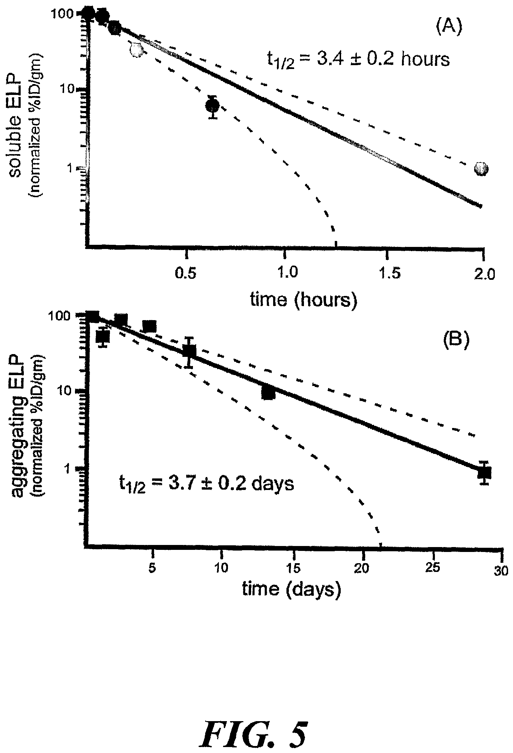

**Figure 5****.** Biodistribution of [¹⁴C]ELP in the injected joint compartment over time. The injected dose (ID/gm) is the amount of ¹⁴C recovered from the injected knee compartment (per gram of recovered tissues and fluids) at time zero (10 min post injection); ID/gm at subsequent time points was normalized by this value to determine a %ID/gm for the knee joint compartment as a function of time. (A) Non-aggregating ELP (Tₜ > 50°C) and (B) aggregating ELP (Tₜ < 35°C). Data expressed as mean ± SE (n=5) were fit to a first-order exponential decay function (solid-line) and plotted on a semi-log axis. Confidence intervals (95%, dashed lines) are also shown. The time constant (τ) for each fit was used to calculate the joint half-life of the two ELP types [t_{1/2} = τ ln(2)).

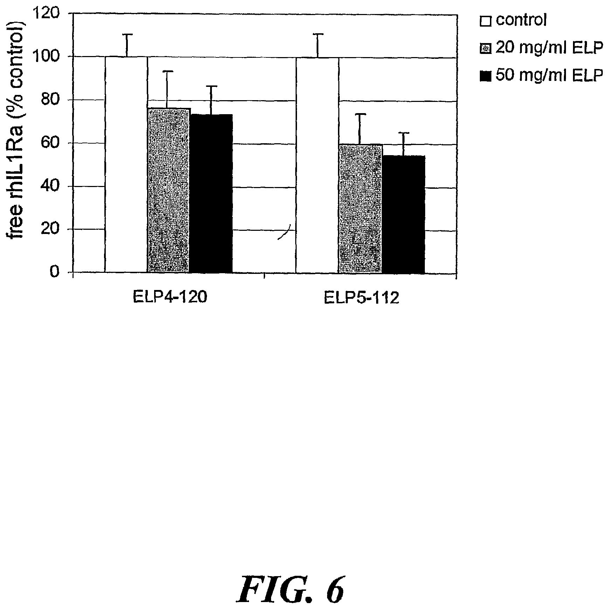

**Figure 6****.** Results for quantifying radiolabeled compound released to solution after mixing with ELP *in vitro.* [³H]rhIL1Ra at 2.5 micrograms/ml was mixed with different ELP formulations of different concentrations at 37°C (ELP4-120 ([(VPGVG)₁₀]₁₂, **SEQ ID NO:2)** and ELP5 ([(VPGVG)₆(VPGKG)]₁₆, **SEQ ID NO:3**))**.** ELP solutions were prepared at concentrations of 0 mg/ml (control), 20, and 50 mg/ml in a total reaction volume of 200 microliters. The ELP-IL1Ra mixture was observed to complex into an aggregate. Aliquots of supernatant (5 microliters) were assayed over time for [³H] via scintillation counting as a measure of rhIL1Ra concentration in the supernatant. Measurements were normalized to control (0 mg/ml, hollow bar) to determine a %free rhIL1Ra in solution = (CPM of sample/CPM of control) x 100. Data are stable within 24h of entrapment; data shown here at 96 hours of equilibration (n=5, mean + SD). Results indicate that the ELPs are able to entrap between 25-55% of this compound upon mixing.

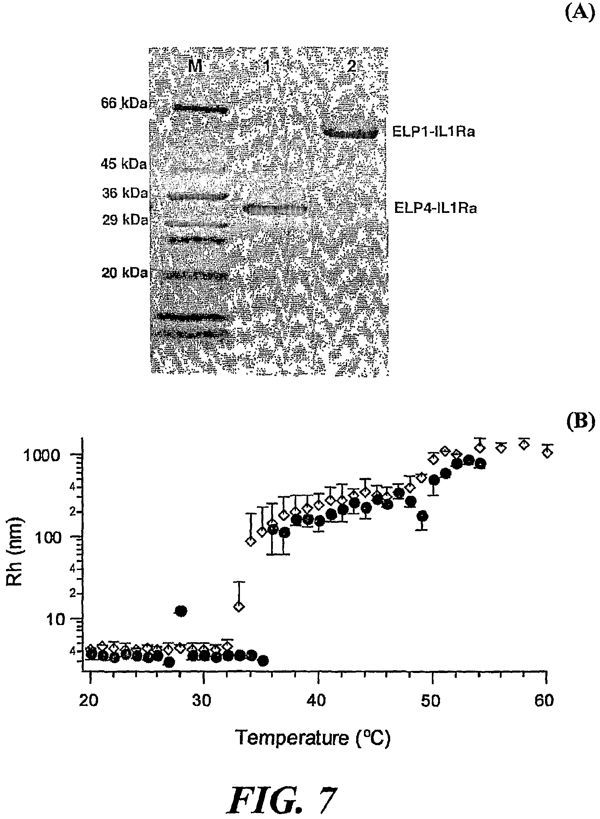

**Figure 7****.** (A) SDS-PAGE of two fusion proteins after two rounds of thermal purification. (B) Aggregation and particle size of the two fusion proteins with temperature were similar, showing the formation of large (> 100 nm) particles beginning at a temperature below 37°C.

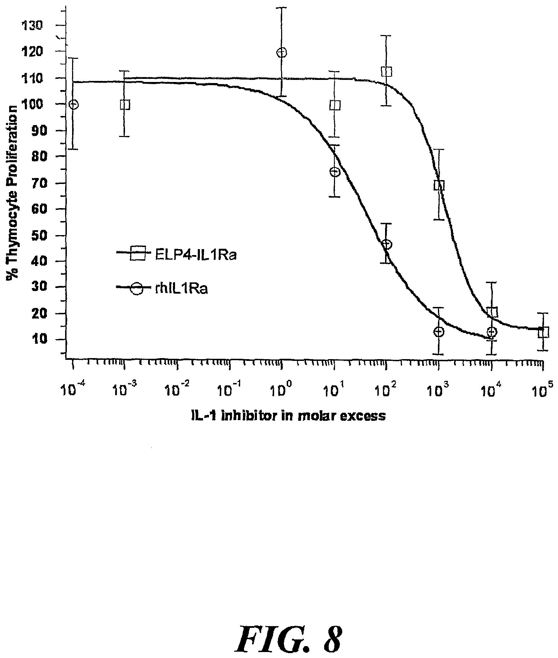

**Figure 8****.** Inhibition of Con A induced IL-1 dependent primary murine thymocytes proliferation by rhIL1Ra or ELP4-IL1Ra fusion protein. Cells cultured for 48 hours with 1 ng/ml murine IL-1β and increasing concentrations of fusion-protein or rhILIRa. Data presented as semi-log plots with the percentage of control proliferation vs. the amount of IL-1 inhibitor added in molar excess over the amount of IL-1β present. Mean ± SD (n=6), data fit to a sigmoid function to determine the IC50 for each protein.

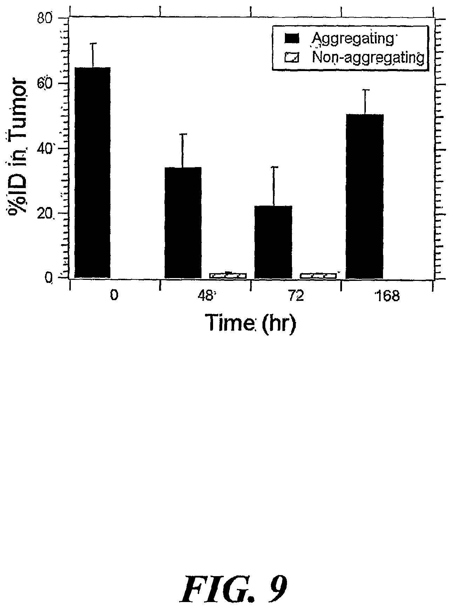

**Figure 9****.** Percent injected dose (%ID) in a tumor of aggregating (solid bar) and non-aggregating (hatched bar) ELPs. Both types of ELPs were infused (4 µL/min) into a human squamous cell carcinoma (FaDu) tumor on the leg of a nude mouse (Balb/c nu/nu). Data are mean ± SEM, n = 1 to 9.

### Detailed Description of the Preferred Embodiments

"Bioelastic polymer" as used herein refers to compounds that comprise repeating elastomeric units. The elastomeric units are typically pentapeptides, tetrapeptides, or nonapeptides. Examples are elastin-like polypeptides or "ELPs".

"Subjects" as used herein includes human subjects (including both male and female subjects and including juvenile, adolescent, adult, and geriatric subjects), as well as animal subjects, particularly other mammalian subjects such as primates, dogs, cats, and horses, for veterinary purposes.

"Arthritis" as used herein means any type of arthritis, including but not limited to rheumatoid arthritis, osteoarthritis, and ankylosing spondylitis.

"Aggregate" as used herein means a collection or agglomeration of a plurality of molecules such that a particle is formed.

"Dis-aggregation" as used herein means a separation of molecule or molecules from an aggregate, a progressive diminishing of aggregate size; or a progressive diminishing of particle size.

"Region of interest" as used herein may be any region of interest, including but not limited to joints and solid tumors ("solid tumor" including the resection cavities within solid tumors after surgical removal of the primary mass thereof and into which the compositions of the invention may be directly administered). In some embodiments of joint administration, the region of interest is an intervetebral disk space or a related spinal joint structure.

"Body temperature" as used herein includes both core body temperature and regional body temperature (*e.g*., the temperature of an extremity when a region of interest such as a joint is located in an extremity).

"Joint" as used herein refers to a movable point where two bones meet, often a synovial joint or syndesmosis, and including but not limited to ball and socket joints, ellipsoidal joints, gliding joints, hinge joints, pivot joints and saddle joints. Such joints may be located in, for example, the shoulder, neck, spine, elbow, hip, wrist, hand, knee, ankle, or foot

"Solid tumor" as used herein may be any solid tumor or cancer, including but not limited to primary and secondary (or metastatic) solid tumors of lung cancer, liver cancer, prostate cancer, ovarian cancer, colon cancer, skin cancer (including melanoma), brain cancer, etc.

"Injection" or "injecting" as used herein may be carried out by any suitable means through a needle, syringe, shunt, cannula (*e.g*., of 7-33 gauge) or any other suitable device that delivers the composition to be delivered directly into the region of interest (in contrast to and not including systemic delivery), as a single bolus or as an infusion over time.

"Antibody" or "antibodies" as used herein refers to all types of immunoglobulins, including IgG, IgM, IgA, IgD, and IgE. The term "immunoglobulin" includes the subtypes of these immunoglobulins, such as IgG₁, IgG₂, IgG₃, IgG₄, etc. Of these immunoglobulins, IgM and IgG are preferred, and IgG is particularly preferred. The antibodies may be of any species of origin, including (for example) mouse, rat, rabbit, horse, or human, or may be chimeric antibodies. The term "antibody" as used herein includes antibody fragments which retain the capability of binding to a target antigen, for example, Fab, F(ab')₂, and Fv fragments, and the corresponding fragments obtained from antibodies other than IgG. Such fragments are also produced by known techniques.

"Chemotherapeutic agent" (or "anticancer agent") as used herein includes but is not limited to methotrexate, daunomycin, mitomycin, cisplatin, vincristine, epirubicin, fluorouracil, verapamil, cyclophosphamide, cytosine arabinoside, aminopterin, bleomycin, mitomycin C, democolcine, etoposide, mithramycin, chlorambucil, melphalan, daunorubicin, doxorubicin, tamosifen, paclitaxel, vincristin, vinblastine, camptothecin, actinomycin D, and cytarabine.

***Compounds of interest.*** Any suitable compound, including but not limited to proteins and peptides, may be coupled to the polymer (*e.g*., via chemical bond or production as a fusion protein) or mixed with the polymer as noted above. In general, the compounds of interest may be detectable groups or a therapeutic group.

For example, the compound of interest may be selected from the group of bone morphogenetic proteins, peptides, and growth factors, more particularly human calcitonin analogs, osteogenic growth peptides, and osteogenic growth peptide-human calcitonin analog hybrids. See, e.g., US Patent No. 6,593,394.

The compound of interest may be selected from the group of antiinfectives such as antibiotics and antiviral agents; chemotherapeutic agents (i.e. anticancer agents); anti-rejection agents; analgesics and analgesic combinations; anti-inflammatory agents; hormones such as steroids; growth factors, including bone morphogenic proteins (i.e. BMP's 1-7), bone morphogenic-like proteins (i.e. GDF-5, GDF-7 and GDF-8), epidermal growth factor (EGF), fibroblast growth factor (i.e. FGF 1-9), platelet derived growth factor (PDGF), insulin like growth factor (IGF-I and IGF-II), transforming growth factors (i.e. TGF-.beta. I-III), vascular endothelial growth factor (VEGF); and other naturally derived or genetically engineered proteins, polysaccharides, glycoproteins, or lipoproteins. See, e.g., D. Overaker, US Patent No. 6,575,986.

The compound of interest may be compounds or agents that actually promote or expedite healing, the effectors may also include compounds or agents that prevent infection (e.g., antimicrobial agents and antibiotics), compounds or agents that reduce inflammation (e.g., anti-inflammatory agents), compounds that prevent or minimize adhesion formation, such as oxidized regenerated cellulose (e.g., INTERCEED™ and SURGICEL™, available from Ethicon, Inc.), hyaluronic acid, and compounds or agents that suppress the immune system (e.g., immunosuppressants). Suitable compounds of interest include heterologous or autologous growth factors, proteins (including matrix proteins), peptides, antibodies, enzymes, platelets, glycoproteins, hormones, cytokines, glycosaminoglycans, nucleic acids, analgesics, viruses, virus particles, and cell types. It is understood that one or more effectors of the same or different functionality may be incorporated within the scaffold. Suitable compounds of interest include the multitude of heterologous or autologous growth factors known to promote healing and/or regeneration of injured or damaged tissue. Suitable compounds of interest include chemotactic agents; therapeutic agents (e.g., antibiotics, steroidal and non-steroidal analgesics and anti-inflammatories, anti-rejection agents such as immunosuppressants and anti-cancer drugs); various proteins (e.g., short term peptides, bone morphogenic proteins, glycoprotein and lipoprotein); cell attachment mediators; biologically active ligands; integrin binding sequence; ligands; various growth and/or differentiation agents and fragments thereof (e.g., epidermal growth factor (EGF), hepatocyte growth factor (HGF), IGF-I, IGF-II, TGF-.beta. I-III, growth and differentiation factors, vascular endothelial growth factors (VEGF), fibroblast growth factors (FGF), platelet derived growth factors (PDGF), insulin derived growth factor (IGF) and transforming growth factors, parathyroid hormone, parathyroid hormone related peptide, bFGF; TGFB superfamily factors; BMP-2; BMP-4; BMP-6; BMP-12; sonic hedgehog; GDF5; GDF6; GDF8; MP52, CDMP1); small molecules that affect the upregulation of specific growth factors; tenascin-C; hyaluronic acid; chondroitin sulfate; fibronectin; decorin; thromboelastin; thrombin-derived peptides; heparin-binding domains; heparin; heparan sulfate; DNA fragments and DNA plasmids. Suitable effectors likewise include the agonists and antagonists of the agents noted above. The growth factor can also include combinations of the growth factors listed above. In addition, the growth factor can be autologous growth factor that is supplied by platelets in the blood. In this case, the growth factor from platelets will be an undefined cocktail of various growth factors. *See, e.g.,* J. Hwang et al., US Patent Application Publication No. 2004/0267362.

In some embodiments, particularly for the treatment of arthritis, the compound of interest is an antiinflammatory agent, examples of which include but are not limited to TNF antibodies, IL-1 antibodies, soluble TNF receptors, soluble IL-1 receptors, TNF receptor antagonists, IL-1 receptor antagonists, COX-2 inhibitors, and non-steroidal antiinflammatory agents (including active fragments thereof for protein or peptide compounds such as antibodies and receptors). Numerous examples of such compounds are known. *See, e.g.,* US Patent No. 6,846,834; *see also* US Patent Nos. 6,624,184; 6,596,746; 6,498,165; and 6,420,373. One particular example is rhIL1Ra (including its isoforms)(commercially available as KINERET™ from Amgen), as described in US Patents Nos. 6,599,873 and 5,075,222.

In the present invention, the composition comprises a therapeutic compound coupled to a polymer as a fusion protein.

***Bioelastic polymers**.* Bioelastic polymers are known and described in, for example, US Patent No. 5,520,672 to Urry et al. In general, bioelastic polymers are polypeptides comprising elastomeric units of bioelastic pentapeptides, tetrapeptides, and/or nonapeptides. Thus in some embodiments the elastomeric unit is a pentapeptide, in other embodiments the elastomeric unit is a tetrapeptide, and in still other embodiments the elastomeric unit is a nonapeptide. Bioelastic polymers that may be used are set forth in U.S. Pat. No. 4,474,851, which describes a number of tetrapeptide and pentapeptide repeating units that can be used to form a bioelastic polymer. Specific bioelastic polymers that can be used are also described in U.S. Pat. Nos. 4,132,746; 4,187,852; 4,500,700; 4,589,882; and 4,870,055. Still other examples of bioelastic polymers are set forth in US Patent No. 6,699,294 to Urry, US Patent No. 6,753,311 to Fertala and Ko, US Patent No. 6,063,061 to Wallace and US Patent No. 6,852,834 to Chilkoti. The bioelastic polymers may contain additional residues or units such as leader and/or trailer sequences as is known in the art.

In one nonlimiting example, the bioelastic polymers are polypeptides that contain at least the general block structure, [(VPGXⁱG)ₙⁱ]ₘ **(SEQ ID NO:4)** for i=1 to J, Xⁱ is any amino acid other than proline (*e.g*., Ala, Leu, Phe), nⁱ is the number of successive repeats of block i adjacent to and preceding block i+1. nⁱ is any number such as 1, 2, 3, or 4 up to 20 and nⁱ are independent of i. J is any number such as 1, 2, or 3 to 5 or more. m is the number of repeats of the block structure where m is any suitable number such as 2, 3 or 4 up to 60, 80 or 300 or more. The identity and frequency of the various amino acids at the fourth position can be changed. For example, the bioelastic polymers may be polypeptides of the general formula: [(VPGAG)₅¹(VPGVG)²(VPGGG)₂³]ₘ, (**SEQ ID NO:5**) where X¹ = A, X² = V, X³ = G, n¹ is 5, n² is 1, n³ is 2, m is at least 1, 2, or 3 up to 100, 150 or 300 or more.

In another nonlimiting example, bioelastic polymers may comprise repeating elastomeric units selected from the group consisting of bioelastic nonpeptides, pentapeptides and tetrapeptides, where the repeating units comprise amino acid residues selected from the group consisting of all amino acid and glycine residues. Preferred amino acid residues are selected from the group consisting of alanine, valine, leucine, isoleucine, proline, phenylalanine, tryptophan, and methionine. In many cases, the first amino acid residue of the repeating unit is a residue of valine, leucine, isoleucine or phenylalanine; the second amino acid residue is a residue of proline; the third amino acid residue is a residue of glycine; and the fourth amino acid residue is glycine or a very hydrophobic residue such as tryptophan, phenylalanine or tyrosine. Particular examples include the tetrapeptide Val-Pro-Gly-Gly (**SEQ ID NO:6**), the tetrapeptide GGVP (**SEQ ID NO:7**), the tetrapeptide GGFP (**SEQ ID NO:8**), the tetrapeptide GGAP (**SEQ ID NO:9**), the pentapeptide is Val-Pro-Gly-Val-Gly (**SEQ ID NO:10**), the pentapeptide GVGVP (**SEQ ID NO:11**), the pentapeptide GKGVP (**SEQ ID NO:12**), the pentapeptide GVGFP (**SEQ ID NO:13**), the pentapeptide GFGFP (**SEQ ID NO:14**), the pentapeptide GEGVP (**SEQ ID NO:15**), the pentapeptide GFGVP (**SEQ ID NO:16**), and the pentapeptide GVGIP (**SEQ ID NO:17**). *See, e.g.,* US Patent No. 6,699,294 to Urry.

The polymer used in the present invention is an Elastin-like polypeptide (ELP) comprising [(VPGVG)5(VPGGG)3(VPGAG)2]9 (SEQ. ID. NO:20).

***Administration.*** As noted above, the composition of the invention is administered to a region of interest by injection in or to the region of interest. The composition comprises the compound to be delivered and a polymer that undergoes an inverse temperature phase transition. The polymer has a transition temperature (Tₜ) less than the body temperature of the subject (*e.g*., less than 37°C). The conjugate aggregates in the region of interest and then gradually disaggregates, providing a sustained release of the compound of interest at the selected region. The administering step is carried out by injection, and particularly intra-articular injection (*e.g.,* into the synovial fluid of a synovial joint).

Patients afflicted with any disease or condition for which a depot drug is desired can be treated with the above methods, with a particular example being patients afflicted with arthritis.

The compound of interest can be administered in any suitable amount depending upon the site of injection, age, weight, and condition of the subject, particular active agent, etc. In general the compound is administered in an amount of from 0.001, 0.01, 0.1, 1, 5 or 10 milligrams per administration or injection, up to about 100, 200 or 300 milligrams per administration or injection. In general the compound is administered at a concentration of 1 pg/ml to 500 mg/ml, with the injection volume dependent upon the region of interest. In general the ELP may also be co-administered at an equivalent concentration of 1 pg/ml to 500 mg/ml, or much higher concentrations in the example of mixing compound with elastomer. In some embodiments the compound of interest preferably has an *in vivo* half life in said region of one or two hours or more; in some embodiments administration of the compound of interest in combination with ELP in the said administration method is associated with an *in vivo* half life for the compound in said region of at least 2 or 3 days, one week, or four weeks or more, up to 2 or three months or more. Stated otherwise, it is contemplated that the administering step will be carried out on a regular and repeated basis (*e.g.,* for the treatment of a chronic condition such as arthritis), but that the frequency of administration will not be greater than, one, two, three or four times per month, while still retaining the desired treatment-effective amount.

The present invention is explained in greater detail in the following non-limiting Examples.

### EXAMPLES

Elastin like polypeptides (ELPs) are genetically engineered biopolymers, made up of the pentapeptide repeat unit as defined above that undergo aggregation, in response to an increase in temperature. ELPs are soluble at temperatures below their transition temperature (Tₜ), and become insoluble and form micron size aggregates at temperatures above their Tₜ. **We disclose herein that a protein drug attached to this drug carrier will aggregate at the time of injection and form a drug *'depot'* at the injection site, which will then slowly disaggregate into the joint space in a sustained manner** (*see, e.g.,* **Figure 1**). In these examples we will evaluate the *in vitro* disaggregation of ELP at a temperature above its Tₜ, as well as the *in vivo* biodistribution and joint half-life of aggregating (Tₜ < 30°C) and non-aggregating (Tₜ > 50°C) ELPs after intra-articular injection. Furthermore, we will design fusion proteins of ELP and IL1Ra and evaluate their binding affinity to the IL-1 receptor and biological activity to inhibit IL-1 to demonstrate proof-of-concept for the ELP fusion protein drug carrier system.

### Reference Example 1

### In Vitro ELP Disaggregation and Peptide Release

Elastin-like polypeptides (ELPs) or bioelastic polymers were evaluated for an ability to provide sustained release of "free" polymer from aggregate form as a proof-of-concept of the proposed delivery system for the treatment of localized diseases, such as osteoarthritis. The drug delivery system utilizes the unique ELP properties of rapid thermally-induced aggregation to form large particles, followed by sustained release of the ELP from the bulk aggregated large particles, termed a "free" form. To evaluate this, two ELP molecules were synthesized utilizing genetic engineering techniques and thermally purified: (1) ELP4-90 = [(VPGVG)₁₀]₉ (**SEQ ID NO:1**), Tₜ = 30.6°C, MW = 37 kDa; and (2) ELP4-120 = [(VPGVG)₁₀]₁₂ (**SEQ ID NO:2**), Tₜ = 28.6°C, MW = 49 kDa, in accordance to known techniques (*see* US Patent No. 6,699, 294 to Urry and US Patent No. 6,852,834 to Chilkoti). For the *in vitro* study, 1.5 ml of either ELP at a concentration of 30 mg/ml was placed in conical tubes and incubated at 37°C for 24h. At the end of this time, the supernatant was completely exchanged for PBS at 37°C and the quantity of ELP in the supernatant quantified as a function of starting ELP (**Figure 2**, t = 0 hours) via absorbance at 280 nm. The sample was returned to 37°C and the amount of free ELP was quantified in 7 µl aliquots of supernatant over time. A time constant for equilibration was determined by numerically fitting data for free ELP concentration to a first-order exponential function.

For both ELP types, the polypeptide underwent an inverse temperature phase transition shortly after being placed at 37°C and slowly reached equilibrium with 15-20% of the starting ELP in a free form (**Figure 2**). The time constants for equilibration were on the order of 1-2 days after the supernatant was completely exchanged. The equilibrium constant describing steady-state values for free to aggregate ELP concentrations were between 0.15-0.20. These results illustrate that an aggregated ELP form will slowly release free ELP over time. In a diarthrodial joint where the free form will be constantly cleared through the synovial tissue, promoting continuous "disaggregation" of the ELP aggregate.

### Reference Example 2

### Biodistribution of ELP After Intra-articular Injection

The biodistribution and joint half-life of an aggregating ELP4-120 ([(VPGVG)₁₀]₁₂, **SEQ ID NO:2,** Tₜ < 32°C, MW 47 kDa) and non-aggregating, ELP2 ([(VPGVG)₁(VPGAG)₈(VPGGG)₇]₁₀, **SEQ ID NO:18,** MW 61 kDa, Tₜ > 50°C). ELPs following intra-articular injection were evaluated in a rat animal model. The ELPs were labeled with [¹⁴C] to yield a specific activity between 32-37 mCi/mmole. The labeled ELP was dialyzed, sterile filtered (0.22 µm filter) and a volume of 30 µl at ∼650 µM of either peptide was injected into the joint cavity of the right knee of Wistar rats. After injection, the animals were housed in a cage for up to 4 weeks. At each time point five rats were sacrificed, their right and left knee (synovial fluid, meniscus, cartilage, synovium), blood, heart, lung, liver, spleen, kidney, and bladder were collected and digested. The radioactivity of each tissue was determined using liquid beta-scintillation counting. Total counts per tissue weight were measured for each animal and for each tissue or body fluid (n=5 animals per group and per time point) and plotted for each tissue as a function of time. Values for joint tissues and joint fluids were summed into a total "joint" value (synovial fluid, meniscus, joint cartilages, synovium).

The total radioactivity recovered from all tissues and fluids in the joint cavity at 10 min after injection was adjusted for injected [¹⁴C]-ELP that was non-specifically distributed, with the starting dose found to be between 70-75% of administered dose. Radioactivity per tissue weight was then normalized by this initial injected knee joint cavity value (10 min post-injection), in order to obtain the percent of injected dose per gram (%ID/gm) of tissue relative to the delivered amount for all recovered tissues and fluids.
At all times, no preferential accumulation of either peptide in the peripheral organs was observed (**Figures 3C** **or** **4C**). Except in the injected knee and blood, the total amount of each peptide was less than 1% ID/gm in all other organs, (Figures **3B** or **4B**). The profile of non-aggregating ELP in the injected knee is shown in **Figure 3** (plotted against time in hours) and that of aggregating ELP in the injected knee is plotted in **Figure 4** (plotted against time in days). The non-aggregating ELP was below 10%ID/gm in the joint space within the first 24 hours, while the aggregating ELP took nearly two weeks to reach the same level.

A radioactive half-life (t_{1/2}) was determined for the injected knee joint compartment by summing the total radioactivity count for the joint as described (**Figure 5**). **Figure 5A** shows injected knee joint compartment values for non-aggregating ELP (Tₜ > 50°C) and 5B showing values for aggregating ELP (Tₜ < 35°C). Data expressed as mean ± SE (n=5) were fit to a first-order exponential decay function (solid-line) and plotted on a semi-log axis. Confidence intervals (95%, dashed lines) are also shown. The time constant (τ) for each fit was used to calculate the joint half-life of the two ELP types [t_{1/2} = τ ln(2)). The half-life of non-aggregating and aggregating ELP in the joint was found to be 3.4 ± 0.2 hours and 3.7 + 0.2 days, respectively. This suggests that the aggregation property of ELP significantly prolongs the half-life of the peptide in articular joints, by over 24 fold (p<0.05, ANOVA), confirming the proposed sustained release mechanism. These findings are significant because they demonstrate that the thermal responsive properties of ELP can be utilized to deliver protein drugs to arthritis-affected joints.

### Reference Example 3

### Demonstration of Entrapment

**Figure 6** demonstrates entrapment by the methods and compositions herein described. **Figure 6** shows results for quantifying radiolabeled compound released to solution after mixing with ELP *in vitro.* [³H]rhIL1Ra at 2.5 micrograms/ml was mixed with different ELP formulations of different concentrations at 37°C (ELP4-120 [(VPGVG)₁₀]₁₂, **SEQ ID NO:2** and ELP5 [(VPGVG)₆(VPGKG)]₁₆, **SEQ ID NO:19**). ELP solutions were prepared at concentrations of 0 mg/ml (control), 20, and 50 mg/ml in a total reaction volume of 200 microliters. The ELP-IL1Ra mixture was observed to complex into an aggregate. Aliquots of supernatant (5 microliters) were assayed over time for [³H] via scintillation counting as a measure of rhILIRa concentration in the supernatant. Measurements were normalized to control (0 mg/ml) to determine a %free rhIL1Ra in solution = (CPM of sample/CPM of control) x 100. Data are stable within 24h of entrapment; data shown here at 96 hours of equilibration. Results indicate that the ELPs are able to entrap between 25-55% of this compound upon mixing.

### Example 4

**(Only the compositions comprising [(VPGVG)5(VPGGG)3(VPGAG)2]9 (SEQ. ID. NO:20) are according to the invention. Compositions comprising other polymers are disclosed for reference purposes).**

### ELP-IL1Ra fusion protein design, synthesis and characterization

In proof-of-concept tests of this drug delivery system, two aggregating fusion proteins of ELP and IL1Ra were genetically synthesized as follows. The gene for human IL1Ra was PCR-cloned from LPS activated U937 cells as previously described by Carter et al. (1990) Nature 344: 633-8. Two ELP formulations were synthesized as described by US Patent No. 6,852,834 to Chilkoti: ELP1 [(VPGVG)₅(VPGGG)₃(VPGAG)₂]₉, (**SEQ ID NO:20**), MW 36 kDa, and ELP4 [(VPGVG)₁₀]₃, (**SEQ ID NO:21**), MW 14.5 kDa. Each ELP gene was then sub-cloned into the IL1Ra containing vector (pET-25b+) using traditional molecular biology techniques to produce two ELP-IL1Ra fusion proteins (see **Tables 1-2**) with molecular weights of 30.2 and 53 kDa. The fusion proteins were then expressed in *E. coli* and thermally purified in accordance with known technique US Patent No. 6,699, 294 to Urry and US Patent No. 6,852,834 to Chilkoti. The size and purity of the fusion proteins was examined by SDS-PAGE and shown in **Figure 7A****.** The thermal purification method used here yielded > 50 mg fusion protein /liter of bacterial growth with > 95% purity.

The temperature driven aggregation of each fusion protein was evaluated by dynamic light scattering, where the scattering of laser light by the fusion protein was monitored while a dilute solution (25 µM) of the fusion protein was heated at 1°C interval over the range of 20 - 60°C. From the scatter data the hydrodynamic radius (Rₕ) was determined by the Stokes-Einstein relationship for spherical particles. The aggregation temperature was defined as the temperature above which large (>100 nm) particles start to form and the fusion protein aggregates. **Figure 7B** shows that the designed fusion proteins undergo temperature driven phase transition and form large aggregates. The aggregates start to form at a temperature below 37°C. This thermal responsive behavior is an important part of the drug delivery system; it implies that the designed fusion proteins of ELP and IL1Ra will aggregate at the time and site of injection, which leads to the increased half-life of the protein drug, ILIRa.

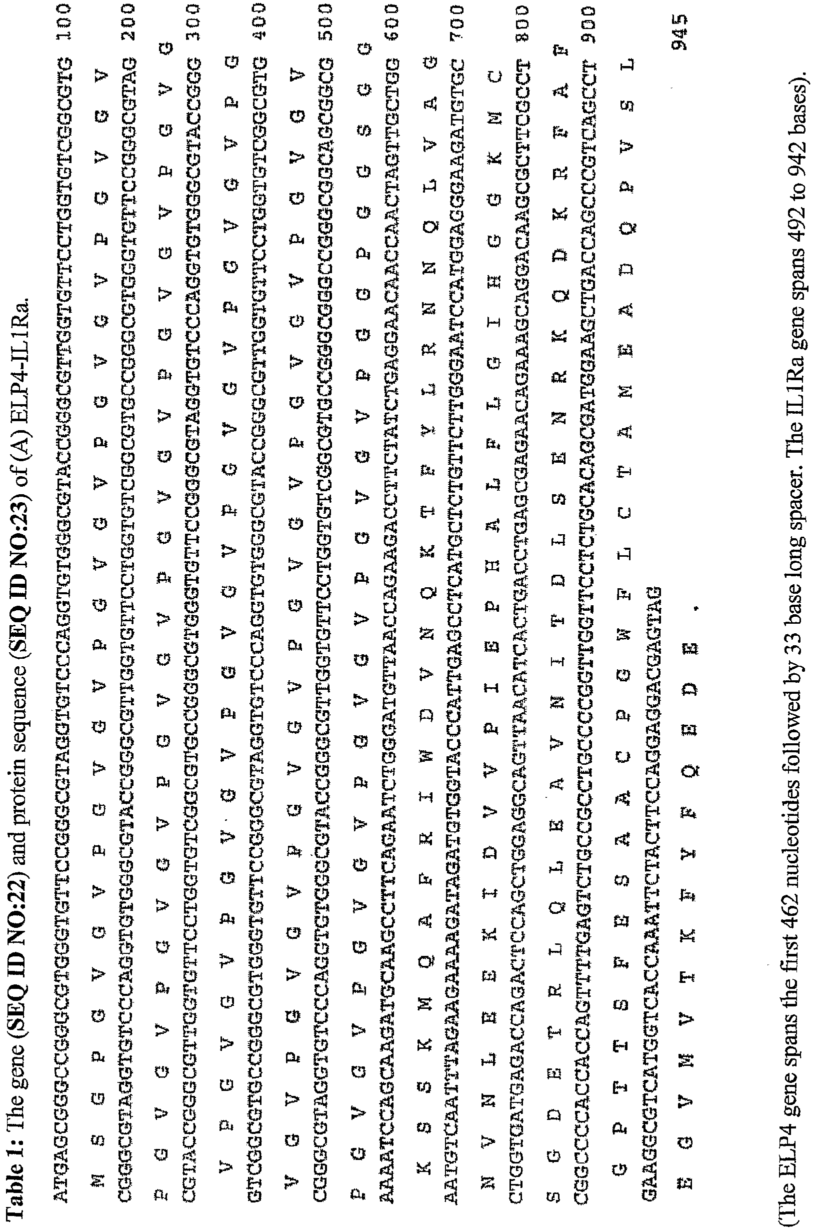

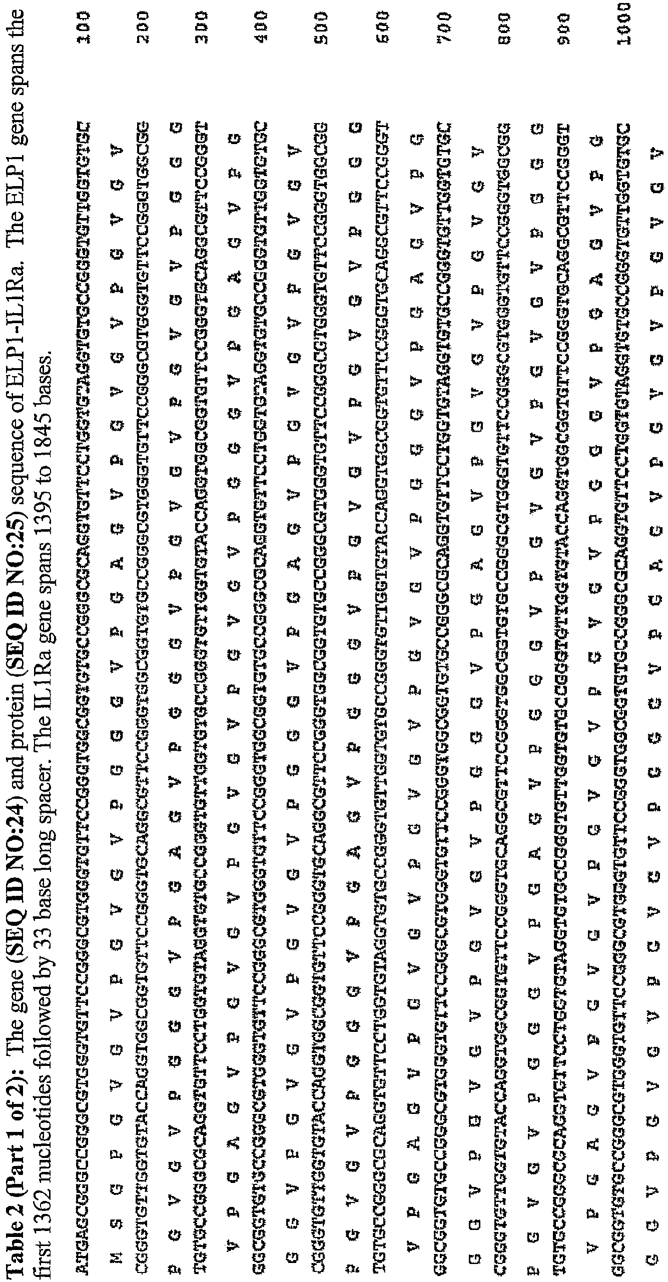

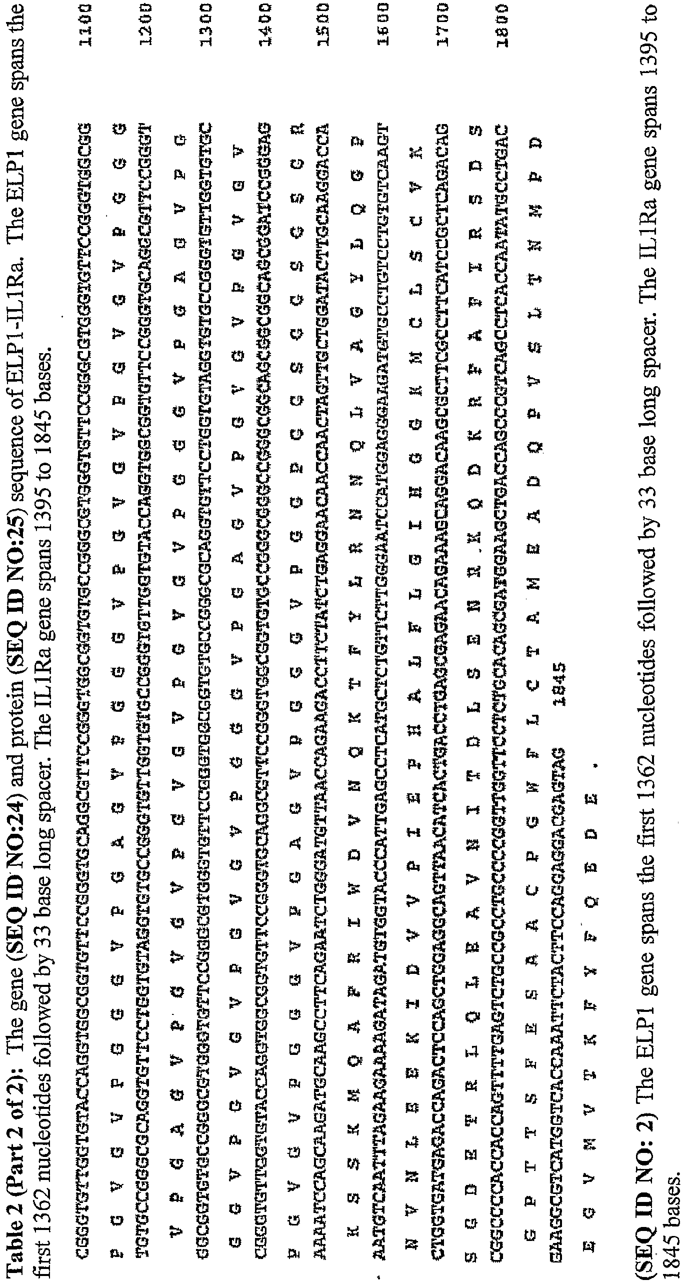

### Reference Example 5

### Biological Activity of ELP-IL1Ra Fusion Protein

The bioactivity of a represententative fusion protein (ELP4-IL1Ra) was examined for its ability to inhibit IL-1β augmented proliferation of Con A-stimulated murine C57 primary thymocytes as previously described. Briefly, primary thymocytes were mechanically isolated from freshly sacrificed 5 to 7 weeks old C57 mice and suspended in RPMI 1640 supplemented with 5 % FCS, 1 µg/ml ConA (Sigma) and 1 ng/ml rmIL-1β (Pierce) at a density of ∼2 x 10⁶ cells/ 200 µl. The cells were then incubated in the presence of serial dilutions of either commercial rhILIRa (R&D Systems) or ELP4-IL1Ra fusion protein (as described above) for 48 hours. The proliferation of thymocytes was assessed by the uptake of [³H]-thymidine (0.5 µCi/well). Data were expressed as mean CPM ± SD of [³H]-thymidine uptake of six replicate cultures. The highest concentration producing 50% inhibition (IC50) was determined by fitting the data to a sigmoid function. The ELP4-ILlRa fusion protein showed significant bioactivity with an IC50 of 61.4 ± 19.8 nM to inhibit 52 pM rmIL-1β, see **Figure 8****.**

### Example 6

**(Only the compositions comprising [(VPGVG)5(VPGGG)3(VPGAG)2]9 (SEQ. ID. NO:20) are according to the invention. Compositions comprising other polymers are disclosed for reference purposes).**

### Tumor administration

Examples of administration of the compositions herein described are given in **Figure 9****.** Percent injected dose (%ID) in a tumor of aggregating (solid bar) and non-aggregating (hatched bar) ELPs is shown. Both types of ELPs were infused (4 µL/min) into a human squamous cell carcinoma (FaDu) tumor on the leg of a nude mouse (Balb/c nu/nu). Data are mean ± SEM, n = 1 to 9. Note the far greater dose for aggregating, as compared to non-aggregating, ELPs.

The foregoing is illustrative of the present invention, and is not to be construed as limiting thereof. The invention is defined by the following claims.

## Claims

1. A pharmaceutically acceptable composition for delivering a drug depot by injection to a selected region in a subject, wherein said composition comprises a therapeutic compound coupled to a polymer as a fusion protein, wherein said polymer undergoes an inverse temperature phase transition and has a transition temperature less than the body temperature of the subject such that a sustained release of said therapeutic compound is provided, and further wherein said polymer is an Elastin-like polypeptide (ELP) comprising [(VPGVG)5(VPGGG)3(VPGAG)2]9 (SEQ. ID. NO:20).

2. A pharmaceutically acceptable composition according to claim 1, wherein said therapeutic compound is an anti-inflammatory protein.

3. A pharmaceutically acceptable composition according to claim 2, wherein said anti-inflammatory protein is an antibody or an antigen-binding antibody fragment.

4. A pharmaceutically acceptable composition according to claim 2, wherein said anti-inflammatory protein is selected from: a TNF blocking antibody; an IL-1 antibody; a soluble TNF receptor; a soluble IL-1 receptor; a TNF receptor antagonist; and an IL-1 receptor antagonist.

5. A pharmaceutically acceptable composition according to claim 4, wherein said anti-inflammatory protein is a recombinant human IL-1 receptor antagonist.

6. A pharmaceutically acceptable composition according to any of claims 1 to 5, wherein the selected region is a joint, synovial joint, joint cavity or an intervertebral disc space.

7. A pharmaceutically acceptable composition according to any of claims 1 to 6, wherein the subject is human.

8. A pharmaceutically acceptable composition comprising a therapeutic compound coupled to a polymer as a fusion protein, wherein said polymer undergoes an inverse temperature phase transition and has a transition temperature less than the body temperature of a subject to be treated such that a sustained release of said therapeutic compound is provided upon injection, and further wherein said polymer is an Elastin-like polypeptide (ELP) comprising [(VPGVG)5(VPGGG)3(VPGAG)2]9 (SEQ. ID. NO:20), for use as a medicament.

9. A pharmaceutically acceptable composition for use according to claim 8, for use in treating a chronic condition, preferably wherein said chronic condition is arthritis, osteoarthritis, diarthrodial joint disorder and/or intervertebral disc pathology, and wherein said composition is administered at a frequency of one, two, three or four times per month.

10. A pharmaceutically acceptable composition for use according to claim 8, wherein said composition is administered at a frequency of one, two, three or four times per month.

11. A pharmaceutically acceptable composition for use according to any of claims 8 to 10, wherein the therapeutic compound is administered in an amount of from 10 milligrams per administration up to 100 milligrams per administration.

12. An injection device comprising a pharmaceutically acceptable composition according to any of claims 1 to 6.

13. An injection device according to claim 12, wherein said device is a needle, syringe, shunt or cannula.

14. An injection device according to claim 13, wherein said needle or cannula is of 7 to 33 gauge.

## Patentansprüche

1. Pharmazeutisch akzeptable Zusammensetzung zur Abgabe eines Wirkstoffdepots durch Injektion an eine ausgewählte Region in einem Probanden, wobei die Zusammensetzung eine therapeutische Verbindung, die mit einem Polymer als ein Fusionsprotein gekoppelt ist, umfasst, wobei das Polymer einen Phasenübergang bei inverser Temperatur erfährt und eine Übergangstemperatur aufweist, die niedriger als die Körpertemperatur des Probanden ist, so dass eine anhaltende Freisetzung der therapeutischen Verbindung bereitgestellt wird, und weiterhin wobei das Polymer ein elastinähnliches Polypeptid (ELP) ist, das [(VPGVG)5(VPGGG)3(VPGAG)2]9 (SEQ ID Nr. 20) umfasst.

2. Pharmazeutisch akzeptable Zusammensetzung nach Anspruch 1, wobei die therapeutische Verbindung ein entzündungshemmendes Protein ist.

3. Pharmazeutisch akzeptable Zusammensetzung nach Anspruch 2, wobei das entzündungshemmende Protein ein Antikörper oder ein antigenbindendes Antikörperfragment ist.

4. Pharmazeutisch akzeptable Zusammensetzung nach Anspruch 2, wobei das entzündungshemmende Protein ausgewählt ist aus: einem TNF-blockierenden Antikörper; einem IL-1-Antikörper; einem löslichen TNF-Rezeptor; einem löslichen IL-1-Rezeptor; einem TNF-Rezeptor-Antagonisten und einem IL-1-Rezeptor-Antagonisten.

5. Pharmazeutisch akzeptable Zusammensetzung nach Anspruch 4, wobei das entzündungshemmende Protein ein rekombinanter humaner IL-1-Rezeptor-Antagonist ist.

6. Pharmazeutisch akzeptable Zusammensetzung nach einem der Ansprüche 1 bis 5, wobei die ausgewählte Region ein Gelenk, ein Synovialgelenk, eine Gelenkhöhle oder ein Bandscheibenfach ist.

7. Pharmazeutisch akzeptable Zusammensetzung nach einem der Ansprüche 1 bis 6, wobei der Proband menschlich ist.

8. Pharmazeutisch akzeptable Zusammensetzung, die eine therapeutische Verbindung, die mit einem Polymer als ein Fusionsprotein gekoppelt ist, umfasst, wobei das Polymer einen Phasenübergang bei inverser Temperatur erfährt und eine Übergangstemperatur aufweist, die niedriger als die Körpertemperatur eines zu behandelnden Probanden ist, so dass bei Injektion eine anhaltende Freisetzung der therapeutischen Verbindung bereitgestellt wird, und weiterhin wobei das Polymer ein elastinähnliches Polypeptid (ELP) ist, das [(VPGVG)5(VPGGG)3(VPGAG)2]9 (SEQ ID Nr. 20) umfasst, zur Verwendung als ein Arzneimittel.

9. Pharmazeutisch akzeptable Zusammensetzung zur Verwendung nach Anspruch 8 zur Verwendung beim Behandeln eines chronischen Leidens, vorzugsweise wobei das chronische Leiden Arthritis, Osteoarthritis, Erkrankung der echten Gelenke und/oder Bandscheibenpathologie ist und wobei die Zusammensetzung in einer Häufigkeit von ein-, zwei-, drei- oder viermal pro Monat verabreicht wird.

10. Pharmazeutisch akzeptable Zusammensetzung zur Verwendung nach Anspruch 8, wobei die Zusammensetzung in einer Häufigkeit von ein-, zwei-, drei- oder viermal pro Monat verabreicht wird.

11. Pharmazeutisch akzeptable Zusammensetzung zur Verwendung nach einem der Ansprüche 8 bis 10, wobei die therapeutische Verbindung in einer Menge von 10 Milligramm pro Verabreichung bis zu 100 Milligramm pro Verabreichung verabreicht wird.

12. Injektionsvorrichtung, die eine pharmazeutisch akzeptable Zusammensetzung nach einem der Ansprüche 1 bis 6 umfasst.

13. Injektionsvorrichtung nach Anspruch 12, wobei die Vorrichtung eine Nadel, eine Spritze, ein Shunt oder eine Kanüle ist.

14. Injektionsvorrichtung nach Anspruch 13, wobei die Nadel oder die Kanüle einen Gauge-Wert von 7 bis 33 aufweist.

## Revendications

1. Composition pharmaceutiquement acceptable pour la délivrance d'un médicament à effet retard par injection dans une région choisie chez un sujet, dans laquelle ladite composition comprend un composé thérapeutique couplé à un polymère sous la forme d'une protéine de fusion, dans laquelle ledit polymère subit une transition de phase en raison inverse de la température et possède une température de transition inférieure à la température corporelle du sujet de façon à obtenir une libération entretenue dudit composé thérapeutique, et en outre dans laquelle ledit polymère est un polypeptide de type élastine (ELP) comprenant [(VPGVG)5(VPGGG)3(VPGAG)2]9 (SEQ. ID. N° 20).

2. Composition pharmaceutiquement acceptable selon la revendication 1, dans laquelle ledit composé thérapeutique est une protéine anti-inflammatoire.

3. Composition pharmaceutiquement acceptable selon la revendication 2, dans laquelle ladite protéine anti-inflammatoire est un anticorps ou un fragment d'anticorps de liaison antigénique.

4. Composition pharmaceutiquement acceptable selon la revendication 2, dans laquelle ladite protéine anti-inflammatoire est choisie parmi : un anticorps de blocage du TNF ; un anticorps anti-IL-1 ; un récepteur soluble du TNF ; un récepteur soluble de l'IL-1 ; un antagoniste du récepteur du TNF ; et un antagoniste du récepteur de l'IL-1.

5. Composition pharmaceutiquement acceptable selon la revendication 4, dans laquelle ladite protéine anti-inflammatoire est un antagoniste recombinant du récepteur de l'IL-1 humaine.

6. Composition pharmaceutiquement acceptable selon l'une quelconque des revendications 1 à 5, dans laquelle la région choisie est une articulation, une articulation synoviale, une cavité articulaire ou un espace de disque intervertébral.

7. Composition pharmaceutiquement acceptable selon l'une quelconque des revendications 1 à 6, dans laquelle le sujet est un être humain.

8. Composition pharmaceutiquement acceptable comprenant un composé thérapeutique couplé à un polymère sous la forme d'une protéine de fusion, dans laquelle ledit polymère subit une transition de phase en raison inverse de la température et possède une température de transition inférieure à la température corporelle d'un sujet à traiter de façon à obtenir une libération entretenue dudit composé thérapeutique lors de l'injection, et en outre dans laquelle ledit polymère est un polypeptide de type élastine (ELP) comprenant [(VPGVG)5(VPGGG)3(VPGAG)2]9 (SEQ. ID. N° 20), destinée à être utilisée comme médicament.

9. Composition pharmaceutiquement acceptable destinée à être utilisée selon la revendication 8, destinée à être utilisée pour traiter une condition chronique, de préférence dans laquelle ladite condition chronique est l'arthrite, l'ostéoarthrite, un trouble d'une articulation diarthrodiale et/ou une pathologie des disques intervertébraux, et dans laquelle ladite composition est administrée à une fréquence d'une, deux, trois ou quatre fois par mois.

10. Composition pharmaceutiquement acceptable destinée à être utilisée selon la revendication 8, dans laquelle ladite composition est administrée à une fréquence d'une, deux, trois ou quatre fois par mois.

11. Composition pharmaceutiquement acceptable destinée à être utilisée selon l'une quelconque des revendications 8 à 10, dans laquelle le composé thérapeutique est administré en une quantité de 10 milligrammes par administration à 100 milligrammes par administration.

12. Dispositif d'injection comprenant une composition pharmaceutiquement acceptable selon l'une quelconque des revendications 1 à 6.

13. Dispositif d'injection selon la revendication 12, dans lequel ledit dispositif est une aiguille, une seringue, une dérivation ou une canule.

14. Dispositif d'injection selon la revendication 13, dans laquelle ladite aiguille ou canule a une gauge de 7 à 33.
